(19) Europäisches Patentamt
European Patent Office
Office européen des brevets

(11) EP 2 083 078 A1

(12) EUROPEAN PATENT APPLICATION

(43) Date of publication:
**29.07.2009 Bulletin 2009/31**

(21) Application number: **09157733.8**

(22) Date of filing: **30.03.2005**

(51) Int Cl.:
***C12N 15/09*** *(2006.01)* ***C12M 1/00*** *(2006.01)*
***C12Q 1/68*** *(2006.01)*

(84) Designated Contracting States:
**AT BE BG CH CY CZ DE DK EE ES FI FR GB GR HU IE IS IT LI LT LU MC NL PL PT RO SE SI SK TR**

(30) Priority: **31.03.2004 JP 2004106136**

(62) Document number(s) of the earlier application(s) in accordance with Art. 76 EPC:
**05728909.2 / 1 731 606**

(71) Applicant: **Tokyo Metropolitan Organization for Medical Research**
**Tokyo 163-8001 (JP)**

(72) Inventors:
- **Ikeda, Kazutaka**
  **Tokyo 168-0081 (JP)**
- **Ide, Soichiro**
  **Hiroshima 737-0142 (JP)**
- **Sora, Ichiro**
  **Miyagi 989-3201 (JP)**

(74) Representative: **HOFFMANN EITLE**
**Patent- und Rechtsanwälte**
**Arabellastrasse 4**
**81925 München (DE)**

Remarks:
This application was filed on 09-04-2009 as a divisional application to the application mentioned under INID code 62.

# (54) Method of evaluating drug sensitivity by analyzing the mu-opioid receptor gene

(57) It is intended to provide a method of evaluating drug sensitivity, which comprises linking a gene polymorphism in the mu-opioid receptor gene or a haplotype composed of the gene polymorphisms to individual drug sensitivity; and an oligonucleotide selected from the group consisting of the nucleotide sequences represented by SEQ ID NOS: 1 to 98.

FIG. 1

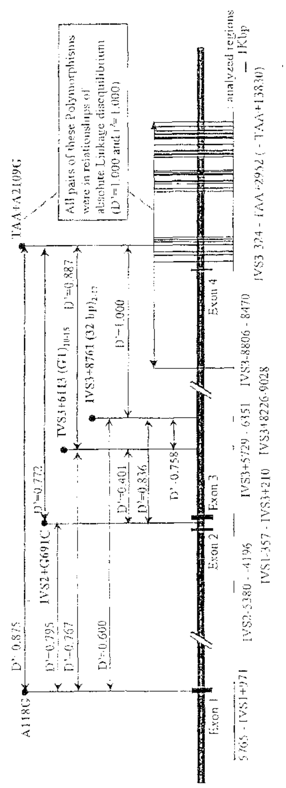

EP 2 083 078 A1

# SEQUENCE LISTING

<110> Tokyo Metropolitan Organization for Medical Research

<120> A method of evaluating drug sensitivity with analyses of mu opioid receptor gene

<130> PCT05-0031

<150> JP2004-106136
<151> 2004-03-31

<160> 98

<170> PatentIn version 3.2

<210> 1
<211> 101
<212> DNA
<213> Homo Sapiens

<400> 1
gttcaactgc taatacctta gcaggaatcg aaacagtgac cccatggcat rctaagagtc 60

actgtactct tcacagacgt gcactcacag aagaaaaaca c 101

<210> 2
<211> 101
<212> DNA
<213> Homo Sapiens

<400> 2
actaaagtag aatgcttgtc ccaaagaaaa gcgcatgttg cctgtttgag ytgtgaacta 60

aattaaccac tttttccgtg gatcactatt tttatttaaa g 101

<210> 3
<211> 101
<212> DNA
<213> Homo Sapiens

<400> 3
atgttgcctg tttgagctgt gaactaaatt aaccactttt tccgtggatc rctattttta 60

tttaaagaat gactgaggcc gggcgcggtg gctcacgcct g 101

<210> 4
<211> 101
<212> DNA
<213> Homo Sapiens

<400> 4
ctgaggccgg gcgcggtggc tcacgcctgt aatcccagca ctttgggagg ytgaggcagg 60

cagatgacga ggtcaggaga tcgagaccat cctggctaac a 101

<210> 5
<211> 101
<212> DNA
<213> Homo Sapiens

<400> 5
actcgggagg tggagcttgc agcgagctga gatcgcgcca ctgcactcca rcctgggcga 60

cagagtgaga ctctgtttta aaataaataa ataaataaaa t 101

<210> 6
<211> 101
<212> DNA
<213> Homo Sapiens

<400> 6
ataaataaaa taaaatataa tgataaagaa atgtttttat agagctctca rttttaattt 60

ctgaagtgat agactgtgat aaagataacc taaataagaa a 101

<210> 7
<211> 101
<212> DNA
<213> Homo Sapiens

<400> 7
taattcttct tgctaatttc taggccacat acaacaggat ataaaaagcc maacaacaaa 60

ggataaattc tttcatatgt gtgtaatcct ataaaccctc t 101

<210> 8
<211> 101
<212> DNA
<213> Homo Sapiens

<400> 8
taaaatatat gctaatcatt ttttcaactg aattcaaata ttatgcacat kaatattcat 60

atatgtttaa tatagaaaga aacacagaga gtgagggagg g 101

<210> 9
<211> 101
<212> DNA
<213> Homo Sapiens

<400> 9
aaaatatatg ctaatcattt tttcaactga attcaaatat tatgcacatt matattcata 60

tatgtttaat atagaaagaa acacagagag tgagggaggg a 101

<210> 10
<211> 101
<212> DNA
<213> Homo Sapiens

<400> 10
ctaatcattt tttcaactga attcaaatat tatgcacatt aatattcata yatgtttaat 60

atagaaagaa acacagagag tgagggaggg agtccactat g 101

<210> 11
<211> 101
<212> DNA
<213> Homo Sapiens

<400> 11
aaaaatctat agtgttgtac tgagctccct ccaaagcaac tataaattta yaggagatga 60

aacatatgat tcaccaggca taagaagaaa gtttccgtaa t 101

<210> 12
<211> 101
<212> DNA
<213> Homo Sapiens

<400> 12
tccacatgaa ctaagcacaa aggaactgaa tgcaggcaga cagatttcag ytcaatataa 60

gagaattgtt acattagttc atggaagaat atgttttaag g 101

<210> 13
<211> 101
<212> DNA
<213> Homo Sapiens

<400> 13
tgtttctcat ttctttttca gaaaataaag gatcgctgtt gttcccaaca kgtttgtagg 60

gaagaaaatt ggagaaacat tattaccttt tcttagatgt t 101

<210> 14
<211> 101
<212> DNA
<213> Homo Sapiens

<400> 14
tagggtttca tcaagccaat gtattccctg ccagatttta aggagaaaaa kgcgctggaa 60

aattgagtga tgttagcccc ctttcttatt tttcactgct a 101

<210> 15
<211> 101
<212> DNA
<213> Homo Sapiens

<400> 15
ccccagcacc cagcccccggt tcctgggtca acttgtccca cttagatggc racctgtccg 60

acccatgcgg tccgaaccgc accgacctgg gcgggagaga c 101

<210> 16
<211> 101
<212> DNA
<213> Homo Sapiens

<400> 16
aatgaaaagg cagaaaaatt agccccaaaa gagatgaaac tcttccgtcc rtcaccattg 60

actctattgt gaacttatga aaaaggtagt tgagcaatat g 101

<210> 17
<211> 101
<212> DNA
<213> Homo Sapiens

<400> 17
gaacttatga aaaaggtagt tgagcaatat gaaggccatg atgtggaatt raacacacac 60

acacacacac acacacacac acacacatgc tggattctaa a 101

<210> 18
<211> 101
<212> DNA
<213> Homo Sapiens

<220>
<221> misc_feature
<222> (51)..(51)
<223> n represents m repeats of ac

<400> 18
acttatgaaa aaggtagttg agcaatatga aggccatgat gtggaattaa natgctggat 60

tctaaaatgt gtccttcctc ctctcactct cttgatcagt t 101

<210> 19
<211> 101
<212> DNA
<213> Homo Sapiens

<400> 19
acagaggtaa tttatttagt ctggcttcac ttaacacaaa taggtcaaaa rcaatcacat 60

tttgtaagta gtaatagttg gagaaatgtg tgaagaatag g 101

<210> 20
<211> 101
<212> DNA
<213> Homo Sapiens

<400> 20
ggtcaaaaga taaataagaa ttattttata accataagaa aggaagaaca kctataaaca 60

aaagtcatat atgcaacata aaagaatagg tgagctgcca g 101

<210> 21
<211> 101
<212> DNA
<213> Homo Sapiens

<400> 21
ttctggaagt tccataaaaa tcactctaat gggtcaaaca tcgatggttc kcagaagaac 60

acaatttttt tcaaaaacga atagcattgt aaattcattt g 101

<210> 22
<211> 101
<212> DNA
<213> Homo Sapiens

<400> 22
tacaacaaaa tacaggcaag gtgagtgatg ttaccagcct gagggaagga rggttcacag 60

cctgatatgt tggtgatgtc ataagcaaag cagtatttat g 101

<210> 23
<211> 101

<212> DNA
<213> Homo Sapiens

<400> 23
tttatatcaa tatagacctc atggaggatc tagctcatgt tgagaggttc rtttttgttc 60

cctgaacgaa agcttaatgt gatcgaagtg gactgcaaaa t 101

<210> 24
<211> 101
<212> DNA
<213> Homo Sapiens

<400> 24
ttccacaatt tctttatagc cttaagttag ctctggtcaa ggctaaaaat saatgagcaa 60

aatggcagta ttaacacctt atgacataat taaatgttgc t 101

<210> 25
<211> 101
<212> DNA
<213> Homo Sapiens

<400> 25
ctctaattac tattattaaa gcactttctt gacattttaa tcaaaatagc rggtcaagaa 60

gttaggagat gctctgtatt tggtttaact gtgaactata t 101

<210> 26
<211> 101
<212> DNA
<213> Homo Sapiens

<400> 26
acatcactct caaaagttga tctcagtttt ttttacaaga catctgtgga ragttaattt 60

gggaaagtaa ttgtttcaat tcaatgggaa aaaaaactca a 101

<210> 27
<211> 101
<212> DNA
<213> Homo Sapiens

<220>
<221> misc_feature
<222> (51)..(51)
<223> n represents 11 to 15 repeats of gt

<400> 27
atcaaaatgg ctattctttc agttctacag tttaaaaaga aaatggttcc ngcgtgtgat 60

ataggcatgt ctctttttgc atgtatggaa ttagagtaaa t 101

<210> 28
<211> 101
<212> DNA
<213> Homo Sapiens

<400> 28
aaagaaaatg gttccgtgtg tgtgtgtgtg tgtgtgtgcg tgtgatatag rcatgtctct 60

ttttgcatgt atggaattag agtaaatgta ggtttaaaat t 101

<210> 29
<211> 101
<212> DNA
<213> Homo Sapiens

<400> 29

tgatatatat cataacatat tatatattat attatgatat atatcataac rtgtattatc 60

atattatgat atatatcata acatatatat tatcatatta c 101

<210> 30
<211> 101
<212> DNA
<213> Homo Sapiens

<400> 30
acatgtatta tcatattatg atatatatca taacatatat attatcatat yacgatatat 60

atcataacat attatatatt atcatattat gatatatatc a 101

<210> 31
<211> 101
<212> DNA
<213> Homo Sapiens

<220>
<221> misc_feature
<222> (51)..(51)
<223> n represents 2 to 17 repeats of attatcatattatgacatatatcataatatat

<400> 31
tatgacatat cataatatat attatcatat tatgacatat cgtaatatat natcaaaaag 60

tcacagagct catgcaagcc cagtcatccc cattgccagt g 101

<210> 32
<211> 101
<212> DNA
<213> Homo Sapiens

<400> 32
aatatatatt atcatattat gacatatatc ataatatata ttatcatatt rtgacatata 60

tcataatata tatcaaaaag tcacagagct catgcaagcc c 101

<210> 33
<211> 101
<212> DNA
<213> Homo Sapiens

<400> 33
taaaatgtac tctttatttc tcactggttt ctccatactg caggctcccc rcatattatt 60

ttcttttttt aactcagctc agaatcctta tgccttttga a 101

<210> 34
<211> 101
<212> DNA
<213> Homo Sapiens

<400> 34
atctaggtag acagccaagt cagatggccc atgcctagaa gctctccatt ytgaactttt 60

gtcagcattg attaaaagaa tcaaatacct tgtagttatc t 101

<210> 35
<211> 101
<212> DNA
<213> Homo Sapiens

<400> 35
cagccaagtc agatggccca tgcctagaag ctctccattt tgaacttttg ycagcattga 60

ttaaaagaat caaatacctt gtagttatct atgatgatac a 101

<210> 36
<211> 101
<212> DNA
<213> Homo Sapiens

<400> 36
ttatgtggac tcaacccacg tatccagtag atgggaaaaa acaaaagcca raataagttt 60

tttagtgttt ccttctgatg aagtttcatg tttgcttgta a 101

<210> 37
<211> 101
<212> DNA
<213> Homo Sapiens

<400> 37
aacaaaagcc aaaataagtt ttttagtgtt tccttctgat gaagtttcat rtttgcttgt 60

aataatctcc atttctcaaa tattatgttc cataatagac a 101

<210> 38
<211> 101
<212> DNA
<213> Homo Sapiens

<400> 38
atgcttttca tgggctagga tggtttctcc caagagatga catagtattg yttttgctca 60

tcaggctgtt tctcagcaat cattgtttct gcttaatacc a 101

<210> 39
<211> 101
<212> DNA
<213> Homo Sapiens

<400> 39
gctcctagta cgaattatct ggcatgttga gagcaacttt gtcttcaagt rggacctgat 60

ctatcttttt ccacaaatgt catgtgtgtg aacaagtttc t 101

<210> 40
<211> 101
<212> DNA
<213> Homo Sapiens

<400> 40
attctaaagt aaataataaa taaggtcatt gtcaacgttt ttcattcaaa rccatttttt 60

aacgtaaatt tgctagaacc accttccaat tccaaggcaa g 101

<210> 41
<211> 101
<212> DNA
<213> Homo Sapiens

<400> 41
taataaataa ggtcattgtc aacgtttttc attcaaaacc atttttaac rtaaatttgc 60

tagaaccacc ttccaattcc aaggcaagga gagacattac a 101

<210> 42
<211> 101
<212> DNA
<213> Homo Sapiens

<400> 42
ctcaactgga tgggctaagg tttctgataa aatctgaaga taaagaaaat sgaatattct 60

gctttttct tccttctaat ttcacccttg cctaaggatg a 101

<210> 43
<211> 101
<212> DNA
<213> Homo Sapiens

<400> 43
tttttcttcc ttctaatttc acccttgcct aaggatgaga tttcttccca sgttggtatc 60

ccagaaatgc agactgtagc tatggggcgg aagctttgtt t 101

<210> 44
<211> 101
<212> DNA
<213> Homo Sapiens

<400> 44
ttgcctaagg atgagatttc ttcccaggtt ggtatcccag aaatgcagac ygtagctatg 60

gggcggaagc tttgtttctt tacctgatca cttgctgtgg a 101

<210> 45
<211> 101
<212> DNA
<213> Homo Sapiens

<400> 45
atttcttccc aggttggtat cccagaaatg cagactgtag ctatggggcg raagctttgt 60

ttctttacct gatcacttgc tgtggaaatt ctagcttatt g 101

<210> 46
<211> 101
<212> DNA

<213> Homo Sapiens

<400> 46
tccctctttc cttgccaatc attagaaagg aaagaagagg aaagagactc kctggagcac 60

tggtgagtct ctaggaccct gctatcctat cccaacaggg c 101

<210> 47
<211> 101
<212> DNA
<213> Homo Sapiens

<400> 47
actggtgagt ctctaggacc ctgctatcct atcccaacag ggctgtcaga mggagaactc 60

ctaatgtggc catttgaaac acttctcaac attgaaatag a 101

<210> 48
<211> 101
<212> DNA
<213> Homo Sapiens

<400> 48
gaagttttaa aataacctct tctaagacac ggctatgagt aggtaagaga kcattcattc 60

ccttcaataa tatgactgtg ttgataaaac tgataaccat t 101

<210> 49
<211> 101
<212> DNA
<213> Homo Sapiens

<400> 49
aactgataac cattcacttg caaatgttat tattgaataa gtctcactta kctcatttaa 60

tattacccaa aagatgctaa caaattctgt ttcccacatt g 101

<210> 50
<211> 101
<212> DNA
<213> Homo Sapiens

<400> 50
gccaaagcaa cctaagaata ggacatggta gcttaagttt ttcagcttct yaactggcca 60

cacacacaca agttgtgttt gtacaattct tgaggtcaat c 101

<210> 51
<211> 101
<212> DNA
<213> Homo Sapiens

<400> 51
caaacaatat tactgtgttc taagcgcttc tgttactcga aaggggtctg rtccagaccc 60

caaaagaggg ttcttggacc tcatgcaaga aagaattcag g 101

<210> 52
<211> 101
<212> DNA
<213> Homo Sapiens

<220>
<221> misc_feature
<222> (51)..(51)
<223> n represents m repeats of a

<400> 52
ggtttgtttt aagtaagcca ctttcctccc tgcaagttcc cacggagcag nggaggaaac 60

tttttcctgg gagcccacta atcacacagt gaacaaaagg c 101

<210> 53
<211> 101
<212> DNA
<213> Homo Sapiens

<400> 53
taagaaagca aaggaataaa gaatggctac tccataggca gcgtagcccc magggctgct 60

ggttggctat ttttgtggtt atttcttgat tatatgctaa a 101

<210> 54
<211> 101
<212> DNA
<213> Homo Sapiens

<400> 54
gtcgctctgg ttcaaacacc tctgacactt gaattacaaa tataaggacc rttgacactg 60

agattttaag ggaggaaaaa cagattgaca gtggactaaa g 101

<210> 55
<211> 101
<212> DNA
<213> Homo Sapiens

<400> 55
gcaaggtaag aatcaagtag aaatgataaa gggcaaggaa aaaagatgaa mgcttactca 60

tattaaccat tctaccattg gaattatttg ccaacacacc t 101

<210> 56

<211> 101
<212> DNA
<213> Homo Sapiens

<400> 56
gacagtgggg aaaattcatc ttcatattgt cacatgcact gtaataggaa kgtttagcaa 60

aaaaaacctt ccagagaaag gtggtttcca atattaccta c 101

<210> 57
<211> 101
<212> DNA
<213> Homo Sapiens

<400> 57
gcaaaaaaaa ccttccagag aaaggtggtt tccaatatta cctacaactt sctttgcaat 60

ttgatttttg aaaggaccta aaagttgaaa acaggctatc a 101

<210> 58
<211> 101
<212> DNA
<213> Homo Sapiens

<220>
<221> misc_feature
<222> (51)..(51)
<223> n represents a sequence having 322bp or deletion

<400> 58
taaatgtttt atttaagttt gcattgccca ctaaggctag acattttttt ngataaattc 60

acagggttac aaaataccaa acggaaatga gataagtggt a 101

<210> 59
<211> 101
<212> DNA
<213> Homo Sapiens

<400> 59
ggcccggcta gacatttttt gataaattca cagggttaca aaataccaaa yggaaatgag 60

ataagtggta taaaccacag aagatatagg agaagagaaa a 101

<210> 60
<211> 101
<212> DNA
<213> Homo Sapiens

<220>
<221> misc_feature
<222> (51)..(51)
<223> n represents a or deletion

<400> 60
tgagataagt ggtataaacc acagaagata taggagaaga gaaaaaaaaa ngaggaaata 60

aagaagacaa ctcttttcct aagagtctgg gtaaaattga a 101

<210> 61
<211> 101
<212> DNA
<213> Homo Sapiens

<400> 61
ggaaataaag aagacaactc ttttcctaag agtctgggta aaattgaaca yagccatatt 60

cactgaacaa catgagtgag cttcattaat ttaagcacag c 101

<210> 62
<211> 101
<212> DNA
<213> Homo Sapiens

<400> 62
ccatattcac tgaacaacat gagtgagctt cattaattta agcacagcaa ractgcttta 60

attaacaaga ccagagagaa gggagaggag actacatttg t 101

<210> 63
<211> 101
<212> DNA
<213> Homo Sapiens

<400> 63
gtgacatatt agacttctta ctttccccaa ataaaaaagt gcctgctggg ygcggtggct 60

cacgcctgta attccagcac tttgggaggc cgaggcgggc g 101

<210> 64
<211> 101
<212> DNA
<213> Homo Sapiens

<400> 64
gcgcggtggc tcacgcctgt aattccagca ctttgggagg ccgaggcggg yggaacacaa 60

ggtcaggaga tcaagaccat cctggccaat atggtaaaac c 101

<210> 65
<211> 101
<212> DNA
<213> Homo Sapiens

<400> 65
atacaaaatt aggaaggcgt ggtggtgcac gcctgtaatc ccagctagtc rggaggctga 60

ggcaggagaa ttgcttgaac tggggaggcg gaagttgcag t 101

<210> 66
<211> 101
<212> DNA
<213> Homo Sapiens

<220>
<221> misc_feature
<222> (51)..(51)
<223> n represents m repeats of a

<400> 66
caagatcgca gcattgcact ccagcctggg caacagaatg agattgtctc ngtgccacat 60

gccatgctat gtgcccaaag tttccttcac acaacacagc c 101

<210> 67
<211> 101
<212> DNA
<213> Homo Sapiens

<400> 67
ttagagccag tcagaattca atctccaata tcctgactag cacaagaaat ycataggttg 60

attcttgttc tcctgcatct ctgcaggtgg caaacctgat t 101

<210> 68
<211> 101
<212> DNA

<213> Homo Sapiens

<400> 68
ttgtgtgttt tcttaataaa ctttacccac ttattaaaag aataaaatga rggtggagtt 60

aattctgact acgggattcc tttttcactt ttataatgaa c 101

<210> 69
<211> 101
<212> DNA
<213> Homo Sapiens

<400> 69
tccttctaac taaatcttat cataagcaaa tctatgcacc aaattattta rtacaattcc 60

taataacagc tgaaggacca tttatttgaa gcaatgttca c 101

<210> 70
<211> 101
<212> DNA
<213> Homo Sapiens

<400> 70
ttagtacaat tcctaataac agctgaagga ccatttattt gaagcaatgt wcaccatagc 60

aaaattccag tgaagtctaa gaactgggac agtccgttga g 101

<210> 71
<211> 101
<212> DNA
<213> Homo Sapiens

<400> 71
ttgccccatg aatgtgcaca tgcatattaa aatatgggca cctcttttaa ktcttttttt 60

tctcataata agtttgaaac tcacagtagg aaattgagag a 101

<210> 72
<211> 101
<212> DNA
<213> Homo Sapiens

<400> 72
tggttgttct cgaactagct ggtttcccag agacagctgg agactgagca mataaagaca 60

tcattgagga aaaaggctac cttgtacctc atggagagct g 101

<210> 73
<211> 101
<212> DNA
<213> Homo Sapiens

<400> 73
catggagagc tgaaggtctg ataaatggga actgccaggt aatagctatg mtatttctga 60

cataaattta aaaactagta ttgtttcttc tagctctgtt t 101

<210> 74
<211> 101
<212> DNA
<213> Homo Sapiens

<220>
<221> misc_feature
<222> (51)..(51)
<223> n represents m repeats of a

<400> 74
taatgttaaa ttggatctat aaacataagt caatttggct ctattatgtc ngagaatagg 60

agttttaact tatatctgtg ttttattaat attttgaagt a 101

<210> 75
<211> 101
<212> DNA
<213> Homo Sapiens

<400> 75
ataagtcaat ttggctctat tatgtcaaaa gagaatagga gttttaactt wtatctgtgt 60

tttattaata ttttgaagta taggaacctc atggtgtagc a 101

<210> 76
<211> 101
<212> DNA
<213> Homo Sapiens

<400> 76
gtatgtgaca ggggctgcat gcaccggtgg tctgggagga acagaacagg rcagggagtt 60

cttctataca atagagaaca gaacaatgtt cttctataca a 101

<210> 77
<211> 101
<212> DNA
<213> Homo Sapiens

<400> 77
aggcgggccc aggcctggtt tcgggcctgg cgctgagctg cctgtatttg rttttacttc 60

cttgttgttt ttactgaata tgaaacaata taaaacaatg t 101

<210> 78

<211> 101
<212> DNA
<213> Homo Sapiens

<400> 78
tggttttact tccttgttgt ttttactgaa tatgaaacaa tataaaacaa kgtgagaggg 60

tctttctctc ctctcaatgt caacatcata tatgattgga g 101

<210> 79
<211> 101
<212> DNA
<213> Homo Sapiens

<400> 79
gctggtttgg ttgaagtttc tcttatcagt caggcacttt gcattttaag ygtactttac 60

caccgacacc ctccccccc agcacacaca cacacacaca c 101

<210> 80
<211> 101
<212> DNA
<213> Homo Sapiens

<400> 80
tcaggcactt tgcattttaa gcgtacttta ccaccgacac cctccccccc magcacacac 60

acacacacac acacacacac acacacaaca tagtgaaatg g 101

<210> 81
<211> 101
<212> DNA
<213> Homo Sapiens

<220>
<221> misc_feature
<222> (51)..(51)
<223> n represents m repeats of ca

<400> 81
ggcactttgc attttaagcg tactttacca ccgacaccct ccccccccag nacatagtga 60

aatggacccg tgggaattat atgatagttg taatcaaaat a 101

<210> 82
<211> 101
<212> DNA
<213> Homo Sapiens

<400> 82
gcactttgca ttttaagcgt actttaccac cgacaccctc cccccccagc rcacacacac 60

acacacacac acacacacac acaacatagt gaaatggacc c 101

<210> 83
<211> 101
<212> DNA
<213> Homo Sapiens

<220>
<221> misc_feature
<222> (51)..(51)
<223> n represents tctc or deletion

<400> 83
tctggaagta aacttaaaat gaaaattaga atttgctttc aattatacta ntatctaaat 60

cttaatttga aatttaaatt attttgtctc tacccaaacc a 101

<210> 84
<211> 101
<212> DNA
<213> Homo Sapiens

<400> 84
tatactatct ctatctaaat cttaatttga aatttaaatt attttgtctc yacccaaacc 60

atcgatttca tggaaatgtt taaattttct tttttttttt t 101

<210> 85
<211> 101
<212> DNA
<213> Homo Sapiens

<400> 85
cttaatttga aatttaaatt attttgtctc tacccaaacc atcgatttca yggaaatgtt 60

taaattttct tttttttttt ttttttgat ggagtctcac t 101

<210> 86
<211> 101
<212> DNA
<213> Homo Sapiens

<220>
<221> misc_feature
<222> (51)..(51)
<223> n represents insertion of tttc or none

<400> 86
tattttgtct ctacccaaac catcgatttc atggaaatgt ttaaattttc nttttttttt 60

ttttttttg atggagtctc actctgtcgc ccaggctgga g 101

<210> 87
<211> 101
<212> DNA
<213> Homo Sapiens

<400> 87
aggctggagt gcagtggctc aatcttggct cactgcaacc tctgcctccc rggttcacac 60

cattctcctg cttcagcctc ctgagtagct gggactacag g 101

<210> 88
<211> 101
<212> DNA
<213> Homo Sapiens

<400> 88
attctcctgc ttcagcctcc tgagtagctg ggactacagg tgcccgccac macacctggc 60

taattttttg tatttttagt agagatgggg tttcaccacg t 101

<210> 89
<211> 101
<212> DNA
<213> Homo Sapiens

<400> 89
acaacacctg gctaattttt tgtattttta gtagagatgg ggtttcacca ygttagccag 60

gatggtttcg atctcctgac ctcgtgatct gcctgcctcg g 101

<210> 90
<211> 101
<212> DNA

<213> Homo Sapiens

<400> 90
atccccatca atttaatagg aattaagtta gaaatactag tatatatatt yccttttatat 60

actaattgta tatccatata aaagcattag taccattata t 101

<210> 91
<211> 101
<212> DNA
<213> Homo Sapiens

<400> 91
agtatatata ttccctttat atactaattg tatatccata taaaagcatt mgtaccatta 60

tatgaaagta tatatgccat tccataaaaa tatatctacc a 101

<210> 92
<211> 101
<212> DNA
<213> Homo Sapiens

<400> 92
ggaattaaag aaaaaatgcc tgttttcact aagtcatcct tcccctggca rtacatttcc 60

tgaactttta catacttaaa tagccagtta tgaaaatgta a 101

<210> 93
<211> 101
<212> DNA
<213> Homo Sapiens

<400> 93
acattttaaa cagactcctg cccacaaact atttttcctc tccaggaata rgaatggcaa 60

ctgaattgtt ccttctttat tctatagctt taagtcaaac c 101

<210> 94
<211> 101
<212> DNA
<213> Homo Sapiens

<400> 94
gaatggcaac tgaattgttc cttctttatt ctatagcttt aagtcaaacc yaacataagc 60

aatcaaccct tccacccatt gtcctctttc tagctgctta t 101

<210> 95
<211> 101
<212> DNA
<213> Homo Sapiens

<400> 95
ttgggggtga aataaaagat agacccctgc tgctctgcac gtagattcag yttgtatgcc 60

agggtgacat tttaatttac agtagtccag acacctaaac a 101

<210> 96
<211> 101
<212> DNA
<213> Homo Sapiens

<400> 96
ggggtgaaat aaaagataga cccctgctgc tctgcacgta gattcagttt statgccagg 60

gtgacatttt aatttacagt agtccagaca cctaaacagg a 101

<210> 97
<211> 101

<212> DNA
<213> Homo Sapiens

<220>
<221> misc_feature
<222> (51)..(51)
<223> n represents m repeats of t

<400> 97
caacattgtt ttccttttga tggtctggga gtttttctat aagtttttgg nctcttcatt 60

agtgtgttag ttccatcatc atgtctgttt actattgaaa a 101

<210> 98
<211> 101
<212> DNA
<213> Homo Sapiens

<400> 98
ttgaaaatat aggcagctaa atccactgat agtctacttt ttttaaaaat ktgttcttga 60

tgttttgagc aggaaaatta tttgcaagaa acaaagagtt t 101

SEQUENCE LISTING

<110> Tokyo Metropolitan Organization for Medical Research

<120> Method of evaluating drug sensitivity by analyzing the mu-opioid receptor gene

<130> 134276

<150> JP2004-106136
<151> 2004-03-31

<160> 98

<170> PatentIn version 3.4

<210> 1
<211> 101
<212> DNA
<213> Homo Sapiens

<400> 1
gttcaactgc taatacctta gcaggaatcg aaacagtgac cccatggcat rctaagagtc 60

actgtactct tcacagacgt gcactcacag aagaaaaaca c 101

<210> 2
<211> 101
<212> DNA
<213> Homo Sapiens

<400> 2
actaaagtag aatgcttgtc ccaaagaaaa gcgcatgttg cctgtttgag ytgtgaacta 60

aattaaccac tttttccgtg gatcactatt tttatttaaa g 101

<210> 3
<211> 101
<212> DNA
<213> Homo Sapiens

<400> 3
atgttgcctg tttgagctgt gaactaaatt aaccactttt tccgtggatc rctattttta 60

tttaaagaat gactgaggcc gggcgcggtg gctcacgcct g 101

<210> 4
<211> 101
<212> DNA
<213> Homo Sapiens

<400> 4
ctgaggccgg gcgcggtggc tcacgcctgt aatcccagca ctttgggagg ytgaggcagg 60

cagatgacga ggtcaggaga tcgagaccat cctggctaac a 101

<210> 5
<211> 101
<212> DNA
<213> Homo Sapiens

```
<400>  5
actcgggagg tggagcttgc agcgagctga gatcgcgcca ctgcactcca rcctgggcga     60

cagagtgaga ctctgtttta aaataaataa ataaataaaa t                       101


<210>  6
<211>  101
<212>  DNA
<213>  Homo Sapiens

<400>  6
ataaataaaa taaaatataa tgataaagaa atgtttttat agagctctca rttttaattt     60

ctgaagtgat agactgtgat aaagataacc taaataagaa a                       101


<210>  7
<211>  101
<212>  DNA
<213>  Homo Sapiens

<400>  7
taattcttct tgctaatttc taggccacat acaacaggat ataaaaagcc maacaacaaa     60

ggataaattc tttcatatgt gtgtaatcct ataaaccctc t                       101


<210>  8
<211>  101
<212>  DNA
<213>  Homo Sapiens

<400>  8
taaaatatat gctaatcatt ttttcaactg aattcaaata ttatgcacat kaatattcat     60

atatgtttaa tatagaaaga aacacagaga gtgagggagg g                       101


<210>  9
<211>  101
<212>  DNA
<213>  Homo Sapiens

<400>  9
aaaatatatg ctaatcattt tttcaactga attcaaatat tatgcacatt matattcata     60

tatgtttaat atagaaagaa acacagagag tgagggaggg a                       101


<210>  10
<211>  101
<212>  DNA
<213>  Homo Sapiens

<400>  10
ctaatcattt tttcaactga attcaaatat tatgcacatt aatattcata yatgtttaat     60

atagaaagaa acacagagag tgagggaggg agtccactat g                       101


<210>  11
<211>  101
<212>  DNA
<213>  Homo Sapiens
```

<400> 11
aaaaatctat agtgttgtac tgagctccct ccaaagcaac tataaattta yaggagatga 60

aacatatgat tcaccaggca taagaagaaa gtttccgtaa t 101

<210> 12
<211> 101
<212> DNA
<213> Homo Sapiens

<400> 12
tccacatgaa ctaagcacaa aggaactgaa tgcaggcaga cagatttcag ytcaatataa 60

gagaattgtt acattagttc atggaagaat atgttttaag g 101

<210> 13
<211> 101
<212> DNA
<213> Homo Sapiens

<400> 13
tgtttctcat ttctttttca gaaaataaag gatcgctgtt gttcccaaca kgtttgtagg 60

gaagaaaatt ggagaaacat tattaccttt tcttagatgt t 101

<210> 14
<211> 101
<212> DNA
<213> Homo Sapiens

<400> 14
tagggtttca tcaagccaat gtattccctg ccagatttta aggagaaaaa kgcgctggaa 60

aattgagtga tgttagcccc ctttcttatt tttcactgct a 101

<210> 15
<211> 101
<212> DNA
<213> Homo Sapiens

<400> 15
ccccagcacc cagccccggt tcctgggtca acttgtccca cttagatggc racctgtccg 60

acccatgcgg tccgaaccgc accgacctgg gcgggagaga c 101

<210> 16
<211> 101
<212> DNA
<213> Homo Sapiens

<400> 16
aatgaaaagg cagaaaaatt agccccaaaa gagatgaaac tcttccgtcc rtcaccattg 60

actctattgt gaacttatga aaaaggtagt tgagcaatat g 101

<210> 17
<211> 101
<212> DNA

<213> Homo Sapiens

<400> 17
gaacttatga aaaaggtagt tgagcaatat gaaggccatg atgtggaatt raacacacac 60

acacacacac acacacacac acacacatgc tggattctaa a 101

<210> 18
<211> 102
<212> DNA
<213> Homo Sapiens

<220>
<221> repeat_region
<222> (51)..(52)
<223> /note="repeats of ac"

<220>
<221> misc_feature
<222> (1)..(102)
<223> /note="see additional information in Table 4, 4-1 and 4-2"

<400> 18
acttatgaaa aaggtagttg agcaatatga aggccatgat gtggaattaa acatgctgga 60

ttctaaaatg tgtccttcct cctctcactc tcttgatcag tt 102

<210> 19
<211> 101
<212> DNA
<213> Homo Sapiens

<400> 19
acagaggtaa tttatttagt ctggcttcac ttaacacaaa taggtcaaaa rcaatcacat 60

tttgtaagta gtaatagttg gagaaatgtg tgaagaatag g 101

<210> 20
<211> 101
<212> DNA
<213> Homo Sapiens

<400> 20
ggtcaaaaga taaataagaa ttattttata accataagaa aggaagaaca kctataaaca 60

aaagtcatat atgcaacata aaagaatagg tgagctgcca g 101

<210> 21
<211> 101
<212> DNA
<213> Homo Sapiens

<400> 21
ttctggaagt tccataaaaa tcactctaat gggtcaaaca tcgatggttc kcagaagaac 60

acaatttttt tcaaaaacga atagcattgt aaattcattt g 101

<210> 22
<211> 101

<212> DNA
<213> Homo Sapiens

<400> 22
tacaacaaaa tacaggcaag gtgagtgatg ttaccagcct gagggaagga rggttcacag 60

cctgatatgt tggtgatgtc ataagcaaag cagtatttat g 101

<210> 23
<211> 101
<212> DNA
<213> Homo Sapiens

<400> 23
tttatatcaa tatagacctc atggaggatc tagctcatgt tgagaggttc rtttttgttc 60

cctgaacgaa agcttaatgt gatcgaagtg gactgcaaaa t 101

<210> 24
<211> 101
<212> DNA
<213> Homo Sapiens

<400> 24
ttccacaatt tctttatagc cttaagttag ctctggtcaa ggctaaaaat saatgagcaa 60

aatggcagta ttaacacctt atgacataat taaatgttgc t 101

<210> 25
<211> 101
<212> DNA
<213> Homo Sapiens

<400> 25
ctctaattac tattattaaa gcactttctt gacattttaa tcaaaatagc rggtcaagaa 60

gttaggagat gctctgtatt tggtttaact gtgaactata t 101

<210> 26
<211> 101
<212> DNA
<213> Homo Sapiens

<400> 26
acatcactct caaaagttga tctcagtttt ttttacaaga catctgtgga ragttaattt 60

gggaaagtaa ttgtttcaat tcaatgggaa aaaaaactca a 101

<210> 27
<211> 130
<212> DNA
<213> Homo Sapiens

<220>
<221> misc_feature
<222> (51)..(80)
<223> /note="11 to 15 repeats of gt"

<220>

```
<221>  repeat_region
<222>  (51)..(80)
<223>  /rpt_type=tandem
       /rpt_unit_seq=gt

<220>
<221>  variation
<222>  (73)..(80)
<223>  /note="Deletion of gt repeat"

<400>  27
atcaaaatgg ctattctttc agttctacag tttaaaaaga aaatggttcc gtgtgtgtgt     60

gtgtgtgtgt gtgtgtgtgt gcgtgtgata taggcatgtc tctttttgca tgtatggaat    120

tagagtaaat                                                           130


<210>  28
<211>  101
<212>  DNA
<213>  Homo Sapiens

<400>  28
aaagaaaatg gttccgtgtg tgtgtgtgtg tgtgtgtgcg tgtgatatag rcatgtctct     60

ttttgcatgt atggaattag agtaaatgta ggtttaaaat t                        101


<210>  29
<211>  101
<212>  DNA
<213>  Homo Sapiens

<400>  29
tgatatatat cataacatat tatatattat attatgatat atatcataac rtgtattatc     60

atattatgat atatatcata acatatatat tatcatatta c                        101


<210>  30
<211>  101
<212>  DNA
<213>  Homo Sapiens

<400>  30
acatgtatta tcatattatg atatatatca taacatatat attatcatat yacgatatat     60

atcataacat attatatatt atcatattat gatatatatc a                        101


<210>  31
<211>  644
<212>  DNA
<213>  Homo Sapiens


<220>
<221>  misc_feature
<222>  (51)..(594)
<223>  /note="2 to 17 repeats of attatcatattatgacatatatcataatatat"

<220>
<221>  repeat_region
<222>  (51)..(594)
```

```
<223>  /rpt_type=tandem
       /rpt_unit_seq=attatcatattatgacatatatcataatatat

<220>
<221>  variation
<222>  (115)..(594)
<223>  /note="Deletion of attatcatattatgacatatatcataatatat repeat"

<400>  31
tatgacatat cataatatat attatcatat tatgacatat cgtaatatat attatcatat      60

tatgacatat atcataatat atattatcat attatgacat atatcataat atatattatc     120

atattatgac atatatcata atatatatta tcatattatg acatatatca taatatatat     180

tatcatatta tgacatatat cataatatat attatcatat tatgacatat atcataatat     240

atattatcat attatgacat atatcataat atatattatc atattatgac atatatcata     300

atatatatta tcatattatg acatatatca taatatatat tatcatatta tgacatatat     360

cataatatat attatcatat tatgacatat atcataatat atattatcat attatgacat     420

atatcataat atatattatc atattatgac atatatcata atatatatta tcatattatg     480

acatatatca taatatatat tatcatatta tgacatatat cataatatat attatcatat     540

tatgacatat atcataatat atattatcat attatgacat atatcataat atatatcaaa     600

aagtcacaga gctcatgcaa gcccagtcat ccccattgcc agtg                     644


<210>  32
<211>  101
<212>  DNA
<213>  Homo Sapiens

<400>  32
aatatatatt atcatattat gacatatatc ataatatata ttatcatatt rtgacatata      60

tcataatata tatcaaaaag tcacagagct catgcaagcc c                         101


<210>  33
<211>  101
<212>  DNA
<213>  Homo Sapiens

<400>  33
taaaatgtac tctttatttc tcactggttt ctccatactg caggctcccc rcatattatt      60

ttcttttttt aactcagctc agaatcctta tgccttttga a                         101


<210>  34
<211>  101
<212>  DNA
<213>  Homo Sapiens

<400>  34
atctaggtag acagccaagt cagatggccc atgcctagaa gctctccatt ytgaactttt      60

gtcagcattg attaaaagaa tcaaatacct tgtagttatc t                         101
```

<210> 35
<211> 101
<212> DNA
<213> Homo Sapiens

<400> 35
cagccaagtc agatggccca tgcctagaag ctctccattt tgaacttttg ycagcattga 60

ttaaaagaat caaatacctt gtagttatct atgatgatac a 101

<210> 36
<211> 101
<212> DNA
<213> Homo Sapiens

<400> 36
ttatgtggac tcaacccacg tatccagtag atgggaaaaa acaaaagcca raataagttt 60

tttagtgttt ccttctgatg aagtttcatg tttgcttgta a 101

<210> 37
<211> 101
<212> DNA
<213> Homo Sapiens

<400> 37
aacaaaagcc aaaataagtt ttttagtgtt tccttctgat gaagtttcat rtttgcttgt 60

aataatctcc atttctcaaa tattatgttc cataatagac a 101

<210> 38
<211> 101
<212> DNA
<213> Homo Sapiens

<400> 38
atgcttttca tgggctagga tggtttctcc caagagatga catagtattg yttttgctca 60

tcaggctgtt tctcagcaat cattgtttct gcttaatacc a 101

<210> 39
<211> 101
<212> DNA
<213> Homo Sapiens

<400> 39
gctcctagta cgaattatct ggcatgttga gagcaacttt gtcttcaagt rggacctgat 60

ctatcttttt ccacaaatgt catgtgtgtg aacaagtttc t 101

<210> 40
<211> 101
<212> DNA
<213> Homo Sapiens

<400> 40
attctaaagt aaataataaa taaggtcatt gtcaacgttt ttcattcaaa rccatttttt 60

aacgtaaatt tgctagaacc accttccaat tccaaggcaa g 101

<210> 41
<211> 101
<212> DNA
<213> Homo Sapiens

<400> 41
taataaataa ggtcattgtc aacgtttttc attcaaaacc attttttaac rtaaatttgc 60

tagaaccacc ttccaattcc aaggcaagga gagacattac a 101

<210> 42
<211> 101
<212> DNA
<213> Homo Sapiens

<400> 42
ctcaactgga tgggctaagg tttctgataa aatctgaaga taaagaaaat sgaatattct 60

gctttttttct tccttctaat ttcacccttg cctaaggatg a 101

<210> 43
<211> 101
<212> DNA
<213> Homo Sapiens

<400> 43
tttttcttcc ttctaatttc acccttgcct aaggatgaga tttcttccca sgttggtatc 60

ccagaaatgc agactgtagc tatggggcgg aagctttgtt t 101

<210> 44
<211> 101
<212> DNA
<213> Homo Sapiens

<400> 44
ttgcctaagg atgagatttc ttcccaggtt ggtatcccag aaatgcagac ygtagctatg 60

gggcggaagc tttgtttctt tacctgatca cttgctgtgg a 101

<210> 45
<211> 101
<212> DNA
<213> Homo Sapiens

<400> 45
atttcttccc aggttggtat cccagaaatg cagactgtag ctatggggcg raagctttgt 60

ttctttacct gatcacttgc tgtggaaatt ctagcttatt g 101

<210> 46
<211> 101
<212> DNA
<213> Homo Sapiens

<400> 46
tccctctttc cttgccaatc attagaaagg aaagaagagg aaagagactc kctggagcac 60

tggtgagtct ctaggaccct gctatcctat cccaacaggg c 101

<210> 47
<211> 101
<212> DNA
<213> Homo Sapiens

<400> 47
actggtgagt ctctaggacc ctgctatcct atcccaacag ggctgtcaga mggagaactc 60

ctaatgtggc catttgaaac acttctcaac attgaaatag a 101

<210> 48
<211> 101
<212> DNA
<213> Homo Sapiens

<400> 48
gaagttttaa aataacctct tctaagacac ggctatgagt aggtaagaga kcattcattc 60

ccttcaataa tatgactgtg ttgataaaac tgataaccat t 101

<210> 49
<211> 101
<212> DNA
<213> Homo Sapiens

<400> 49
aactgataac cattcacttg caaatgttat tattgaataa gtctcactta kctcatttaa 60

tattacccaa aagatgctaa caaattctgt ttcccacatt g 101

<210> 50
<211> 101
<212> DNA
<213> Homo Sapiens

<400> 50
gccaaagcaa cctaagaata ggacatggta gcttaagttt ttcagcttct yaactggcca 60

cacacacaca agttgtgttt gtacaattct tgaggtcaat c 101

<210> 51
<211> 101
<212> DNA
<213> Homo Sapiens

<400> 51
caaacaatat tactgtgttc taagcgcttc tgttactcga aaggggtctg rtccagaccc 60

caaaagaggg ttcttggacc tcatgcaaga aagaattcag g 101

<210> 52
<211> 101
<212> DNA
<213> Homo Sapiens

<220>
<221> repeat_region

<222> (51)..(51)
<223> /note="repeats of a"

<220>
<221> misc_feature
<222> (1)..(101)
<223> /note="see additional information in Table 4, 4-1 and 4-2"

<400> 52
ggtttgtttt aagtaagcca ctttcctccc tgcaagttcc cacggagcag aggaggaaac 60

tttttcctgg gagcccacta atcacacagt gaacaaaagg c 101

<210> 53
<211> 101
<212> DNA
<213> Homo Sapiens

<400> 53
taagaaagca aaggaataaa gaatggctac tccataggca gcgtagcccc magggctgct 60

ggttggctat ttttgtggtt atttcttgat tatatgctaa a 101

<210> 54
<211> 101
<212> DNA
<213> Homo Sapiens

<400> 54
gtcgctctgg ttcaaacacc tctgacactt gaattacaaa tataaggacc rttgacactg 60

agattttaag ggaggaaaaa cagattgaca gtggactaaa g 101

<210> 55
<211> 101
<212> DNA
<213> Homo Sapiens

<400> 55
gcaaggtaag aatcaagtag aaatgataaa gggcaaggaa aaaagatgaa mgcttactca 60

tattaaccat tctaccattg gaattatttg ccaacacacc t 101

<210> 56
<211> 101
<212> DNA
<213> Homo Sapiens

<400> 56
gacagtgggg aaaattcatc ttcatattgt cacatgcact gtaataggaa kgtttagcaa 60

aaaaaacctt ccagagaaag gtggtttcca atattaccta c 101

<210> 57
<211> 101
<212> DNA
<213> Homo Sapiens

<400> 57
gcaaaaaaaa ccttccagag aaaggtggtt tccaatatta cctacaactt sctttgcaat 60

ttgatttttg aaaggaccta aaagttgaaa acaggctatc a 101

<210> 58
<211> 422
<212> DNA
<213> Homo Sapiens

<220>
<221> variation
<222> (51)..(372)
<223> /replace=''''

<400> 58
taaatgtttt atttaagttt gcattgccca ctaaggctag acattttttt tttttttttt 60

tttttttttt tttttgagac agagtctcgc tctgtcgccc aggctggagt acagtggcgg 120

gatctcggct cactgcaagc tccgcctccc gggttcacgc cattctcctg cctcagcctc 180

ccaagtagct gggactacag gcgcccgcca ctacgcccgg ctaatttttt gtatttttag 240

tagagacggg gtttcaccgt tttagccggg atgatctcga tctcctgacc tcgtgatccg 300

cccgcctcgg cctcccaaag tgctgggatt acaggcgtga gccaccgcgc ccggcccggc 360

tagacatttt ttgataaatt cacagggtta caaaatacca aacggaaatg agataagtgg 420

ta 422

<210> 59
<211> 101
<212> DNA
<213> Homo Sapiens

<400> 59
ggcccggcta gacatttttt gataaattca cagggttaca aaataccaaa yggaaatgag 60

ataagtggta taaaccacag aagatatagg agaagagaaa a 101

<210> 60
<211> 101
<212> DNA
<213> Homo Sapiens

<220>
<221> variation
<222> (51)..(51)
<223> /replace=''''

<400> 60
tgagataagt ggtataaacc acagaagata taggagaaga gaaaaaaaaa agaggaaata 60

aagaagacaa ctcttttcct aagagtctgg gtaaaattga a 101

<210> 61
<211> 101
<212> DNA
<213> Homo Sapiens

<400> 61
ggaaataaag aagacaactc ttttcctaag agtctgggta aaattgaaca yagccatatt 60

cactgaacaa catgagtgag cttcattaat ttaagcacag c 101

<210> 62
<211> 101
<212> DNA
<213> Homo Sapiens

<400> 62
ccatattcac tgaacaacat gagtgagctt cattaattta agcacagcaa ractgcttta 60

attaacaaga ccagagagaa gggagaggag actacatttg t 101

<210> 63
<211> 101
<212> DNA
<213> Homo Sapiens

<400> 63
gtgacatatt agacttctta ctttccccaa ataaaaaagt gcctgctggg ygcggtggct 60

cacgcctgta attccagcac tttgggaggc cgaggcgggc g 101

<210> 64
<211> 101
<212> DNA
<213> Homo Sapiens

<400> 64
gcgcggtggc tcacgcctgt aattccagca ctttgggagg ccgaggcggg yggaacacaa 60

ggtcaggaga tcaagaccat cctggccaat atggtaaaac c 101

<210> 65
<211> 101
<212> DNA
<213> Homo Sapiens

<400> 65
atacaaaatt aggaaggcgt ggtggtgcac gcctgtaatc ccagctagtc rggaggctga 60

ggcaggagaa ttgcttgaac tggggaggcg gaagttgcag t 101

<210> 66
<211> 101
<212> DNA
<213> Homo Sapiens

<220>
<221> repeat_region
<222> (51)..(51)
<223> /note="repeats of a"

<220>
<221> misc_feature
<222> (1)..(101)

<223> /note="see additional information in Table 4, 4-1 and 4-2"

<400> 66
caagatcgca gcattgcact ccagcctggg caacagaatg agattgtctc agtgccacat 60

gccatgctat gtgcccaaag tttccttcac acaacacagc c 101

<210> 67
<211> 101
<212> DNA
<213> Homo Sapiens

<400> 67
ttagagccag tcagaattca atctccaata tcctgactag cacaagaaat ycataggttg 60

attcttgttc tcctgcatct ctgcaggtgg caaacctgat t 101

<210> 68
<211> 101
<212> DNA
<213> Homo Sapiens

<400> 68
ttgtgtgttt tcttaataaa ctttacccac ttattaaaag aataaaatga rggtggagtt 60

aattctgact acgggattcc tttttcactt ttataatgaa c 101

<210> 69
<211> 101
<212> DNA
<213> Homo Sapiens

<400> 69
tccttctaac taaatcttat cataagcaaa tctatgcacc aaattattta rtacaattcc 60

taataacagc tgaaggacca tttatttgaa gcaatgttca c 101

<210> 70
<211> 101
<212> DNA
<213> Homo Sapiens

<400> 70
ttagtacaat tcctaataac agctgaagga ccatttattt gaagcaatgt wcaccatagc 60

aaaattccag tgaagtctaa gaactgggac agtccgttga g 101

<210> 71
<211> 101
<212> DNA
<213> Homo Sapiens

<400> 71
ttgccccatg aatgtgcaca tgcatattaa aatatgggca cctcttttaa ktcttttttt 60

tctcataata agtttgaaac tcacagtagg aaattgagag a 101

<210> 72
<211> 101

<212> DNA
<213> Homo Sapiens

<400> 72
tggttgttct cgaactagct ggtttcccag agacagctgg agactgagca mataaagaca 60

tcattgagga aaaaggctac cttgtacctc atggagagct g 101

<210> 73
<211> 101
<212> DNA
<213> Homo Sapiens

<400> 73
catggagagc tgaaggtctg ataaatggga actgccaggt aatagctatg mtatttctga 60

cataaattta aaaactagta ttgtttcttc tagctctgtt t 101

<210> 74
<211> 101
<212> DNA
<213> Homo Sapiens

<220>
<221> repeat_region
<222> (51)..(51)
<223> /note="repeats of a"

<220>
<221> misc_feature
<222> (1)..(101)
<223> /note="see additional information in Table 4, 4-1 and 4-2"

<400> 74
taatgttaaa ttggatctat aaacataagt caatttggct ctattatgtc agagaatagg 60

agttttaact tatatctgtg ttttattaat attttgaagt a 101

<210> 75
<211> 101
<212> DNA
<213> Homo Sapiens

<400> 75
ataagtcaat ttggctctat tatgtcaaaa gagaatagga gttttaactt wtatctgtgt 60

tttattaata ttttgaagta taggaacctc atggtgtagc a 101

<210> 76
<211> 101
<212> DNA
<213> Homo Sapiens

<400> 76
gtatgtgaca ggggctgcat gcaccggtgg tctgggagga acagaacagg rcagggagtt 60

cttctataca atagagaaca gaacaatgtt cttctataca a 101

<210> 77

<211> 101
<212> DNA
<213> Homo Sapiens

<400> 77
aggcgggccc aggcctggtt tcgggcctgg cgctgagctg cctgtatttg rttttacttc 60

cttgttgttt ttactgaata tgaaacaata taaaacaatg t 101

<210> 78
<211> 101
<212> DNA
<213> Homo Sapiens

<400> 78
tggttttact tccttgttgt ttttactgaa tatgaaacaa tataaaacaa kgtgagaggg 60

tctttctctc ctctcaatgt caacatcata tatgattgga g 101

<210> 79
<211> 101
<212> DNA
<213> Homo Sapiens

<400> 79
gctggtttgg ttgaagtttc tcttatcagt caggcacttt gcattttaag ygtactttac 60

caccgacacc ctcccccccc agcacacaca cacacacaca c 101

<210> 80
<211> 101
<212> DNA
<213> Homo Sapiens

<400> 80
tcaggcactt tgcattttaa gcgtacttta ccaccgacac cctccccccc magcacacac 60

acacacacac acacacacac acacacaaca tagtgaaatg g 101

<210> 81
<211> 102
<212> DNA
<213> Homo Sapiens

<220>
<221> repeat_region
<222> (51)..(52)
<223> /note="repeats of ca"

<220>
<221> misc_feature
<222> (1)..(102)
<223> /note="see additional information in Table 4, 4-1 and 4-2"

<400> 81
ggcactttgc attttaagcg tactttacca ccgacaccct cccccccag caacatagtg 60

aaatggaccc gtgggaatta tatgatagtt gtaatcaaaa ta 102

<210> 82
<211> 101
<212> DNA
<213> Homo Sapiens

<400> 82
gcactttgca ttttaagcgt actttaccac cgacaccctc cccccccagc rcacacacac 60

acacacacac acacacacac acaacatagt gaaatggacc c 101

<210> 83
<211> 104
<212> DNA
<213> Homo Sapiens

<220>
<221> variation
<222> (51)..(54)
<223> /replace=''''

<400> 83
tctggaagta aacttaaaat gaaaattaga atttgctttc aattatacta tctctatcta 60

aatcttaatt tgaaatttaa attattttgt ctctacccaa acca 104

<210> 84
<211> 101
<212> DNA
<213> Homo Sapiens

<400> 84
tatactatct ctatctaaat cttaatttga aatttaaatt attttgtctc yacccaaacc 60

atcgatttca tggaaatgtt taaattttct tttttttttt t 101

<210> 85
<211> 101
<212> DNA
<213> Homo Sapiens

<400> 85
cttaatttga aatttaaatt attttgtctc tacccaaacc atcgatttca yggaaatgtt 60

taaattttct tttttttttt tttttttgat ggagtctcac t 101

<210> 86
<211> 100
<212> DNA
<213> Homo Sapiens

<220>
<221> variation
<222> (50)..(51)
<223> /note="possible insertion of tttc"

<400> 86
tattttgtct ctacccaaac catcgatttc atggaaatgt ttaaattttc tttttttttt 60

tttttttga tggagtctca ctctgtcgcc caggctggag 100

<210> 87
<211> 101
<212> DNA
<213> Homo Sapiens

<400> 87
aggctggagt gcagtggctc aatcttggct cactgcaacc tctgcctccc rggttcacac 60

cattctcctg cttcagcctc ctgagtagct gggactacag g 101

<210> 88
<211> 101
<212> DNA
<213> Homo Sapiens

<400> 88
attctcctgc ttcagcctcc tgagtagctg ggactacagg tgcccgccac macacctggc 60

taattttttg tatttttagt agagatgggg tttcaccacg t 101

<210> 89
<211> 101
<212> DNA
<213> Homo Sapiens

<400> 89
acaacacctg gctaattttt tgtattttta gtagagatgg ggtttcacca ygttagccag 60

gatggtttcg atctcctgac ctcgtgatct gcctgcctcg g 101

<210> 90
<211> 101
<212> DNA
<213> Homo Sapiens

<400> 90
atccccatca atttaatagg aattaagtta gaaatactag tatatatatt ycctttatat 60

actaattgta tatccatata aaagcattag taccattata t 101

<210> 91
<211> 101
<212> DNA
<213> Homo Sapiens

<400> 91
agtatatata ttccctttat atactaattg tatatccata taaaagcatt mgtaccatta 60

tatgaaagta tatatgccat tccataaaaa tatatctacc a 101

<210> 92
<211> 101
<212> DNA
<213> Homo Sapiens

<400> 92
ggaattaaag aaaaaatgcc tgttttcact aagtcatcct tcccctggca rtacatttcc 60

```
tgaactttta catacttaaa tagccagtta tgaaaatgta a                         101


<210>  93
<211>  101
<212>  DNA
<213>  Homo Sapiens

<400>  93
acattttaaa cagactcctg cccacaaact atttttcctc tccaggaata rgaatggcaa      60

ctgaattgtt ccttctttat tctatagctt taagtcaaac c                         101


<210>  94
<211>  101
<212>  DNA
<213>  Homo Sapiens

<400>  94
gaatggcaac tgaattgttc cttctttatt ctatagcttt aagtcaaacc yaacataagc      60

aatcaaccct tccacccatt gtcctctttc tagctgctta t                         101


<210>  95
<211>  101
<212>  DNA
<213>  Homo Sapiens

<400>  95
ttgggggtga aataaaagat agacccctgc tgctctgcac gtagattcag yttgtatgcc      60

agggtgacat tttaatttac agtagtccag acacctaaac a                         101


<210>  96
<211>  101
<212>  DNA
<213>  Homo Sapiens

<400>  96
ggggtgaaat aaaagataga cccctgctgc tctgcacgta gattcagttt statgccagg      60

gtgacatttt aatttacagt agtccagaca cctaaacagg a                         101


<210>  97
<211>  101
<212>  DNA
<213>  Homo Sapiens


<220>
<221>  repeat_region
<222>  (51)..(51)
<223>  /note="repeats of t"

<220>
<221>  misc_feature
<222>  (1)..(101)
<223>  /note="see additional information in Table 4, 4-1 and 4-2"

<400>  97
caacattgtt ttccttttga tggtctggga gtttttctat aagtttttgg tctcttcatt      60
```

```
agtgtgttag ttccatcatc atgtctgttt actattgaaa a                        101


<210>  98
<211>  101
<212>  DNA
<213>  Homo Sapiens

<400>  98
ttgaaaatat aggcagctaa atccactgat agtctacttt ttttaaaaat ktgttcttga     60

tgttttgagc aggaaaatta tttgcaagaa acaaagagtt t                        101
```

## Description

### Technical Field

The present invention relates to a method of evaluating drug sensitivity by analyzing a mu-opioid receptor gene.

### Background Art

Stimulants such as methamphetamine exert its action by activating monoamine neurotransmission, and it is considered that in particular dopamine is most directly involved in an action of reinforcing a stimulant (Document 1). It is generally well known that the dopamine nervous system and the opioid nervous system interact with each other. Heretofore, it has been reported that an opioid receptor agonist regulates dopamine metabolism in the nerve terminal to induce dopamine release to a synaptic cleft (Document 2), and it attenuates methamphetamine-induced alterations in dopamine neurotransmission (Documents 3 and 4). Further, in the recent studies using knockout animals and the like, it is shown that there is a close interaction between both nervous systems (Documents 5 to 7). Therefore, it is considered that alterations in the opioid receptor function may cause individual differences in susceptibility to stimulant addiction and stimulant-induced psychotic-like symptoms.

On the other hand, it has been revealed that a mu-opioid receptor plays a very important role for an endogenous opioid peptide or a narcotic analgesic agent to develop an analgesic action and various side effects such as development of addiction, development of resistance and withdrawal symptoms. Further, recently, it became known that a mu-opioid receptor is also involved in addiction to a drug other than narcotic analgesic agents or alcohol or a part of mental diseases (Documents 8 to 11).

In particular, it is believed that a mu-opioid receptor gene polymorphism may have an influence on induction or suppression of development of addiction caused by an opioid drug or a nonopioid drug. Heretofore, in studies on mu-opioid receptor gene polymorphisms, the existence of polymorphisms mainly in translation regions, 5'UTR and a part of introns is shown using genes mainly of Europeans and Americans (Caucasian African-American) (Documents 12 to 14). In particular, as for a single nucleotide polymorphism A118G that causes an amino acid mutation, there is a report that the binding affinity of β-endarphin, which is one of endogenous ligands, increases in a mutant form of receptor which has been expressed in a cultured cell system, which has attracted attention. Further, mainly A118G has been investigated for narcotic or alcohol addiction in various races, however, the presence or absence of correlation varies depending on researchers, and the present situation is that a unified conclusion has not been achieved yet (Documents 15 to 21). On the other hand, a number of studies on the relationship between genetic factors of diseases or pathologic conditions and human gene polymorphisms have been carried out. However, many of them were analyses carried out using genomes of Europeans and Americans. It is known that the presence or absence of gene polymorphism and occurrence frequency greatly vary depending on races, therefore, it is essential that an analysis be carried out using Japanese genomes upon studying Japanese diseases or pathologic conditions and genetic factors.

### Documents

1. Uhl GR, Hall FS, Sora I. Cocaine, reward, movement and monoamine transporters. Mol Psychiatry, 7, 21-6, (2002)
2. Wood PL. Opioid regulation of CNS dopaminergic pathways: a review of methodology, receptor types, regional variations and species differences. Peptides, 4, 595-601, (1983)
3. El Daly E, Chefer V, Sandill S, Shippenberg TS. Modulation of the neurotoxic effects of methamphetamine by the selective kappa-opioid receptor agonist U69593. J Neurochem, 74, 1553-62, (2000)
4. Hayashi T, Tsao LI, Cadet JL, Su TP. [D-Ala2, D-Leu5]enkephalin blocks the methamphetamineinduced c-fos mRNA increase in mouse stratum. Eur J Pharmacol, 366, R7-8, (1999)
5. Smith JW, Fetsko LA, Xu R, Wang Y. Dopamine D2L receptor knockout mice display deficits in positive and negative reinforcing properties of morphine and in avoidance learning. Neuroscience, 113, 755-65, (2002)
6. Spielewoy C, Gonon F, Roubert C, Fauchey V, Jaber M, Caron MG, et al. Increased rewarding properties of morphine in dopamine-transporter knockout mice. Eur J Neurosci, 12, 1827-37, (2000)
7. Chefer VI, Kieffer BL, Shippenberg TS. Basal and morphine-evoked dopaminergic neurotransmission in the nucleus accumbens of MOR- and DCR-knockout mice. Eur J Neurosci, 18, 1915-22, (2003)
8. Lichtman AH, Sheikh SM, Loh HH, Martin BR. Opioid and cannabinoid modulation of precipitated withdrawal in delta(9)-tetrahydrocannabinol and morphine-dependent mice. J Pharmacol Exp Ther, 298, 1007-14, (2001)
9. Becker A, Grecksch G, Kraus J, Loh HH, Schroeder H, Hollt V. Rewarding effects of ethanol and cocaine in mu opioid receptor-deficient mice. Naunyn Schmiedebergs Arch Pharmacol, 365, 296-302, (2002)

10. Berrendero F, Kieffer BL, Maldonado R. Attenuation of nicotine-induced antinociception, rewarding effects, and dependence in mu-opioid receptor knock-out mice. J Neurosci, 22, 10935-40, (2002)
11. Contarino A, Picetti R, Matthes HW, Koob GF, Kieffer BL, Gold LH. Lack of reward and locomotor stimulation induced by heroin in mu-opioid receptor-deficient mice. Eur J Pharmacol, 446, 103-9, (2002)
12. Uhl GR, Sora I, Wang Z. The mu opiate receptor as a candidate gene for pain: polymorphisms, variations in expression, nociception, and opiate responses. Proc Natl Acad Sci U S A, 96, 7752-5, (1999)
13. Hoehe MR, Kopke K, Wendel B, Rohde K, Flachmeier C, Kidd KK, et al. Sequence variability and candidate gene analysis in complex disease: association of mu opioid receptor gene variation with substance dependence. Hum Mol Genet, 9, 2895-908, (2000)
14. Mayer P, Hollt V. Allelic and somatic variations in the endogenous opioid system of humans. Pharmacol Ther, 91, 167-77, (2001)
15. Bond C, LaForge KS, Tian M, Melia D, Zhang S, Borg L, et al. Single-nucleotide polymorphism in the human mu opioid receptor gene alters beta-endorphin binding and activity: possible implications for opiate addiction. Proc Natl Acad Sci U S A, 95, 9608-13, (1998)
16. Bergen AW, Kokoszka J, Peterson R, Long JC, Virkkunen M, Linnoila M, et al. Mu opioid receptor gene variants: lack of association with alcohol dependence. Mol Psychiatry, 2, 490-4, (1997)
17. Sander T, Gscheidel N, Wendel B, Samochowiec J', Smolka M, Rommelspacher H, et al. Human mu-opioid receptor variation and alcohol dependence. Alcohol Clin Exp Res, 22, 2108-10, (1998)
18. Gelernter J, Kranzler H, Cubells J. Genetics of two mu opioid receptor gene (OPRM1) exon I polymorphisms: population studies, and allele frequencies in alcohol-and drug-dependent subjects. Mol Psychiatry, 4, 476-83, (1999)
19. Franke P, Wang T, Nothen MM, Knapp M, Neidt H, Albrecht S, et al. Nonreplication of association between mu-opioid-receptor gene (OPRM1) A118G polymorphism and substance dependence. Am J Med Genet, 105, 114-9, (2001)
20. Shi J, Hui L, Xu Y, Wang F, Huang W, Hu G. Sequence variations in the mu-opioid receptor gene (OPRM1) associated with human addiction to heroin. Hum Mutat, 19, 459-60, (2002)
21. Schinka JA, Town T, Abdullah L, Crawford FC, Ordorica PI, Francis E, et al. A functional polymorphism within the mu-opioid receptor gene and risk for abuse of alcohol and other substances. Mol Psychiatry, 7, 224-8, (2002)

Disclosure of the invention

An object of the present invention is to provide a method of predicting individual differences in susceptibility to development of methamphetamine addiction from which many patients suffer among stimulant addictions and stimulant-induced psychotic-like symptoms using a mu-opioid receptor gene polymorphism.

The inventor made intensive studies and as a result, found that several novel mu-opioid receptor gene polymorphisms and elucidated that the gene polymorphisms are different between methamphetamine-addicted patients and normal subjects, thus the present invention has been completed.

That is, the present invention is as follows.

(1) A method of evaluating drug sensitivity, which comprises linking a gene polymorphism in a mu-opioid receptor gene or a haplotype comprising the gene polymorphism to individual drug sensitivity.
In the present invention, as the above-mentioned gene polymorphisms, at least one polymorphism selected from the group consisting of single nucleotide polymorphisms (SNP), insertion polymorphisms, deletion polymorphisms and nucleotide repeat polymorphisms. Further, as the above-mentioned gene polymorphisms, polymorphisms shown in Table 4 can be exemplified. Further, the above-mentioned haplotype is, for example, a haplotype shown in Table 5 or 8.
As the above-mentioned drug, a mu-opioid receptor function modulator, for example, at least one member selected from the group consisting of methamphetamine methylenedioxymethamphetamine, amphetamine, dextroamphetamine, dopamine, morphine, DAMGO, codeine, methadone, carfentanil, fentanyl, heroin, cocaine, naloxone, naltrexone, nalorphine, levallorphan, pentazocine, buprenorphine, oxycodone, hydrocodone, levorphanol, etorphine, dihydroetorphine, hydromorphone, oxymorphone, ethanol, methanol, diethyl ether and tramadol can be exemplified.
(2) A method of determining a type and/or an amount of a drug to be administered to an individual by using as an index, a result evaluated by the above-mentioned method.
(3) A method of predicting a side effect of a drug to be administered to an individual by using as an index, a result evaluated by the above-mentioned method.
(4) An oligonucleotide comprising a sequence of at least 10 bases containing the 51st base in a nucleotide sequence represented by any of SEQ ID NOS: 1 to 98 or a sequence complementary to this.
Preferably, the above-mentioned oligonucleotide has a length of 10 to 45 bases containing the 51st base.
(5) An oligonucleotide selected from the group consisting of the nucleotide sequences represented by SEQ ID NOS:

1 to 98 and nucleotide sequences complementary to these.
(6) A microarray, in which the above-mentioned oligonucleotide has been immobilized on a support.
(7) A kit for evaluating drug sensitivity including the above-mentioned oligonucleotide and/or the above-mentioned microarray.

According to the present invention, a novel mu-opioid receptor gene polymorphism is provided. By using the polymorphism of the present invention, individual differences in sensitivity to a drug can be evaluated. By this method of evaluating sensitivity, an appropriate prescribed amount of a narcotic drug such as morphine can be found.

Brief Description of the Drawings

Fig. 1 is a schematic view of a mu-opioid receptor gene (an analyzed range and results of linkage disequilibrium analysis among major polymorphisms).

Best Mode for Carrying Out the Invention

Hereinafter, the present invention will be described in detail.

1. Outline of the present invention

The present inventor identified many novel gene polymorphisms by analyzing mu-opioid receptor gene polymorphisms for normal subjects. The human mu-opioid receptor mRNAs are encoded by exons 1 to 4. The 5' untranslated region is present upstream of exon 1. Introns 1 to 3 are present between exons 1 and 2, 2 and 3, and 3 and 4, respectively, and the 3' untranslated region is present downstream of exon 4. As for the gene polymorphisms of the present invention, a number of novel polymorphisms different from the published polymorphisms are contained in these introns, exons and the like. Many of the SNPs found in intron 3 and the 3' untranslated region among the novel gene polymorphisms of the present invention are in complete linkage disequilibrium. Here, the linkage equilibrium means a case where the relationship between two polymorphisms on the chromosome is independent, and the linkage disequilibrium means a case where a polymorphism is linked to the other polymorphism thereby deviating from the equilibrium situation according to Mendel's law of independence.

Further, the present inventor systematized the information of a group of SNPs according to haplotypes by using the above-mentioned SNP information. The haplotype means a genetic structure of such as genes or SNPs located in the vicinity of each other in one allele of a set of alleles (a gene derived from one of the parents). SNPs located in the vicinity on a genome are inherited in a block of strong linkage disequilibrium (a haplotype block). In other words, a haplotype also refers to a combination of the arrangement of the same gene in this haplotype block. In the case where several SNPs appear in association with a certain phenotype (for example, occurrence of a disease), even if not all the SNPs are typed, by analyzing several SNPs constituting a haplotype, a relationship between the genotype and the phenotype of a patient can be elucidated. The present inventor could estimate four haplotype blocks from the results of the haplotype analysis.

Disease occurrence or drug sensitivity is not associated with only one polymorphism, but is associated on a haplotype unit in many cases. Further, it is very difficult to elucidate an unknown polymorphism present outside the untranslated region. Therefore, a haplotype is used for finding a relationship between a polymorphism and a disease or drug sensitivity. Accordingly, the present invention also provides a method using not only the above-mentioned SNP but also a haplotype as a gene polymorphism to be used for evaluating drug sensitivity.

The present inventor compared the SNP frequencies typified by the four estimated haplotype blocks elucidated from the results of the haplotype analysis between normal subjects and methamphetamine-addicted patients, and found that there was a difference in the occurrence frequencies between both groups, and in particular, the difference between both groups was evident when comparison was carried out after performing classification according to stimulant-induced psychotic-like symptoms.

Accordingly, by analyzing SNPs or haplotypes elucidated in the present invention, it becomes possible to evaluate individual drug sensitivity. This drug sensitivity will be important information upon determining the type or amount of a drug. In particular, a stimulant or morphine may cause a big social problem depending on the usage, therefore, it is extremely useful to know in advance an appropriate amount of a drug to be administered to an individual before administering the drug.

2. Mu-opioid receptor gene polymorphism

The human mu-opioid receptor gene polymorphism of the present invention include mainly a single nucleotide polymorphism (SNP), an insertion/deletion polymorphism, and a polymorphism caused by the difference in the number of repeats of base sequence.

The SNP (SNPs) means a polymorphism caused by replacing a specific one base of a gene with another base. The insertion/deletion polymorphism means a polymorphism caused by deletion/insertion of one or more bases. Further, the polymorphism caused by the difference in the number of repeats of base sequence (nucleotide repeat polymorphism) include a microsatellite polymorphism (the number of bases: about 2 to 4 bases) and a VNTR (variable number of tandem repeat) polymorphism (repeated bases: several to several tens of bases) according to the difference in the number of repeated bases, and means a polymorphism in which the number of repeats varies depending on individuals.

The information of human mu-opioid receptor gene polymorphisms elucidated by the present invention is shown in the following Table 1, Table 2 and Table 3. In these tables, single nucleotide polymorphisms, insertion/ deletion polymorphisms, and polymorphisms caused by the difference in the number of repeats of base sequence are included.

The present invention provides an oligonucleotide containing a gene polymorphism shown in Table 1, Table 2 and Table 3 among mu-opioid receptor genes. In the present invention, a method of obtaining gene polymorphism information shown in Table 1, Table 2 and Table 3 is as follows.

Genomic DNA is purified from a blood specimen collected from a human using the phenol method and the like. At this time, a commercially available genomic DNA extraction kit such as GFX Genomic Blood DNA Purification Kit or a device may be used. Then, the genomic DNA is divided into several regions and amplified by the PCR method using the resulting genomic DNA as a template, thereby to prepare a template DNA for sequencing. The present invention is directed to a gene polymorphism, therefore, it is preferred that an enzyme with as higher fidelity as possible is used in the PCR method. As for the 5' untranslated region (5' UTR and 5' flanking region) of the mu-opioid receptor, a region up to 6000 bp, preferably 5800 bp upstream from the start codon is divided into 2 to 4 regions, and PCR amplification is carried out. As for exon 1, exon 2-intron 2-exon 3, exon 4, and a part of intron 1 or 3, the full length is amplified by the PCR method for each region. The exon 4 may contain a region of about 3000 bp downstream from the stop codon. As for the 3' untranslated region, a region up to 14000 bp, preferably 13900 bp downstream from the stop codon is divided into 4 to 6 regions, and amplification is carried out by PCR. The nucleotide sequences of the full regions of these PCR fragments are analyzed such that a segment of about 500 bp was decoded by the sequencing method at a time using primers designed based on the sequence information published in GenBank, whereby a gene polymorphism of interest can be obtained. The thus obtained polymorphism information is shown in Table 1.

Table 1: SNP found on genome of Japanese normal subjects and polymorphism frequencies thereof

| Position | SNP name | Amino acid substitution | Allelic frequency | Sample size | Reported Allelic frequency |
|---|---|---|---|---|---|
| 5'flanking region and 5'UTR | A-55800 | | <1.5% | 44 | |
| | C-5342T | | <1.5% | 44 | |
| | A-5308G | | <1.5% | 44 | |
| | C-5236T | | <1.5% | 44 | |
| | G-5026A | | <1.5% | 44 | |
| | G-4936A | | <1.5% | 44 | |
| | C-4504A | | <1.5% | 44 | |
| | T-2694G | | <1.5% | 44 | |
| | A-2693C | | <1.5% | 44 | |
| | T-2683C | | <1.5% | 44 | |
| | T-2402C | | <1.5% | 44 | |
| | C-1609T | | <1.5% | 44 | |
| | G-696T | | <1.5% | 44 | |
| | G-490T | | <1.5% | 44 | |
| Exon 1 | A118G | Asn40Asp | 45.3% | 213 | 7.5-25.8% |
| Intron 1 | IVS1-A4980G | - | 2.7% | 187 | |
| | IVS1-A4910G | - | 2.7% | 187 | |
| | IVS1-G4690A | - | <1.5% | 113 | |
| | IVS1-T4591G | - | 2.7% | 113 | |
| | IVS1-T4456G | - | <1.5% | 113 | |
| Intron 2 | IVS2+G31A | - | 2.6% | 232 | 4.2% |

(continued)

| Position | SNP name | Amino acid substitution | Allelic frequency | Sample size | Reported Allelic frequency |
|---|---|---|---|---|---|
| | IVS2+G518A | - | <1.5% | 232 | |
| | IVS2+G691C | - | 81.9% | 232 | 42.5 - 53.3% |
| Intron 3 | IVS3+G5807A | - | 44.4% | 179 | |
| | IVS3+G5953A | - | 13.1% | 179 | |
| | IVS3+A6151G | - | 92.5% | 179 | |
| | IVS3+A8449G | - | 9.2% | 179 | |
| | IVS3+C8497T | - | 31.3% | 179 | |
| | IVS3-G8804A | - | 9.5% | 179 | |
| 3'UTR | TAA+G886A | - | 10.8% | 97 | |
| | TAA+T1360C | - | <1.5% | 97 | |
| | TAA+T1371C | - | 10.8% | 97 | |
| | TAA+G1670A | - | 11.4% | 44 | |
| | TAA+G1709A | - | . 5.7% | 44 | |
| | TAA+C2007T | - | 11.4% | 44 | |
| | TAA+A2109G | - | 8.4% | 179 | |
| | TAA+A2274G | - | 11.7% | 179 | |
| | TAA+G2287A | - | 90.8% | 179 | |
| | TAA+G2395C | - | 8.4% | 179 | |

In Table 1, "Position" means a position on the genome of a mu-opioid gene, and indicates 5' or 3' untranslated region, exon or intron. "SNP name" is the name of SNP at a position on the genome, and given by the present inventor. Basically, an alphabet of A, G, C or T is given before and after tree- or four-digit number and symbol, respectively, thus which base is involved in SNP can be identified. For example, "A-5580G" indicates a polymorphism in which the base 5580 bp upstream (5' side) of the start codon is changed between A and G. "IVS1-A4980G" in the column "Intron-1" indicates a polymorphism in intron 1, in which, assuming that the base which is in intron 1 and in contact with exon 2 is 1, the base 4980 bp upstream thereof has been changed from A to G. In the same manner, "TAA+G886A" in the column "3' UTR" indicates a polymorphism in which the base located at the 3' side of 886 bp from a stop codon, TAA, has been changed from G to A.

Table 2: Polymorphism found in 3' untranslated region of mu-opioid receptor gene and linkage disequilibrium

| TAA+ | reported | major allele | minor allele | LD | TAA+ | reported | major allele | minor allele | LD |
|---|---|---|---|---|---|---|---|---|---|
| 886 | A | G | A | * | 8386 | A(13) | A(16) | A(13) | |
| 1360 | T | T | C | | 9000 | C | C | T | |
| 1371 | T | T | C | * | 9564 | A | G | | |
| 1670 | A | G | A | * | 9669 | G | A | G | * |
| 1709 | G | G | A | | 9716 | T | T | A | |
| 2007 | C | C | T | * | 9839 | T | G | T | * |
| 2026 | G | G | A | | 9994 | C | A | C | * |
| 2109 | A | A | G | * | 10083 | C | C | A | |
| 2274 | A | A | G | | 10223 | A(4) | A(3) | A(4) | * |
| 2287 | G | A | G | * | 10247 | A | T | A | * |
| 2395 | G | G | C | (*) | 10535 | A | G | | |
| 2458 | G | G | C | | 10704 | G | G | A | |
| 2482 | T | T | C | | 10752 | T | G | T | * |

(continued)

| TAA+ | reported | major allele | minor allele | LD | TAA+ | reported | major allele | minor allele | LD |
|---|---|---|---|---|---|---|---|---|---|
| 2497 | G | G | A | | 11100 | C | T | | |
| 2656 | G | G | T | * | 11129 | C | A | C | * |
| 2714 | C | A | | | 11132 | CA(17) | CA(17) | CA(14) | * |
| 2820 | G | G | T | * | 11133 | A | A | G | |
| 2907 | G | G | T | | 11368 | TCTC | - | TCTC | * |
| 3423 | T | C | | | 11411 | T | T | C | |
| 4026 | A | G | | | 11431 | T | C | T | * |
| 4585 | A(6) | A(5) | A(6) | | 11449 | - | TITC | - | * |
| 4861 | A | A | C | | 11541 | G | G | A | * |
| 5359 | A | A | G | | 11602 | A | C | A | * |
| 6074 | A | A | C | | 11650 | C | T | | |
| 6866 | T | T | G | * | 11918 | C | C | T | * |
| 6922 | C | G | C | * | 11956 | A | C | | |
| 7075-7396 | 322 bp | - | | | 12143 | A | A | G | * |
| 7427 | C | C | T | * | 12630 | A | G | | |
| 7483 | A | - | | | 12681 | T | T | C | * |
| 7536 | T | C | T | * | 12831 | T | C | | |
| 7589 | A | G | G | | 12834 | G | C | | |
| 8116 | C | T | C | * | 13236 | T(15) | T(14) | T(15) | |
| 8165 | C | T | T | | 13971 | G | T | | |
| 8281 | G | A | A | | | | | | |

The distance indicates the number of bases from a stop codon (TAA). "reported" indicates a genotype in a genebank sequence, "major" and "minor" indicate a major genotype and minor genotype on the Japanese genome, respectively. The numerical value in a parenthesis in the table means the number of repeats of the base before the numerical value, and "-" means deletion or insertion. * in the column of LD (Linkage disequilibrium) indicates a polymorphism belonging to a group of polymorphisms which were in significant and complete linkage disequilibrium (D' = 1.000, $r^2$ = 1.000), and (*) indicates a polymorphism which was in significant linkage disequilibrium (D' = 1.000).

SNP in Table 2 indicates a polymorphism in the 3' untranslated region. The column of "TAA+" indicates the number of bases from a stop codon, TAA, in the mu-opioid receptor. "reported" indicates a genotype of a sequence registered in GenBank, and "major allele" and "minor allele" indicate a major genotype and minor genotype on the Japanese genome, respectively. The value in a parenthesis indicates the number of repeats of the base before the value. For example, "A(6)" means that A is repeated 6 times. "-" means deletion or insertion. "*" in the column of "LD" (Linkage disequilibrium) means that it is in significant and complete linkage disequilibrium (D' = 1. 000, $r^2$ = 1.000).

Table 3 shows examples of nucleotide repeat polymorphisms of the mu-opioid receptor gene in normal subjects.

Table 3: Nucleotide repeat polymorphism of mu-opioid receptor gene in normal subject

| | | | | | | | | | | | | | |
|---|---|---|---|---|---|---|---|---|---|---|---|---|---|
| | number of repeats | 11 | 12 | 13 | 14 | 15 | | | | | | | |
| IVS3+6113 | control (2n=358) (GT)n<br>(%) | 35<br>(9.8) | 1<br>(0.3) | 284<br>(79.3) | 33<br>(9.2) | 5<br>(1.4) | | | | | | | |
| | number of repeats | 2 | - | 8 | 9 | 10 | 11 | 12 | 13 | 14 | 15 | 16 | 17 |
| IVS3+8761<br>(32bp)n | control (2n=358)<br>(%) | 34<br>(9.5) | - | 1<br>(0.3) | 12<br>(3.4) | 100<br>(27.9) | 33<br>(9.2) | 118<br>(33.0) | 18<br>(5.0) | 9<br>(2.5) | 6<br>(1.7) | 25<br>(7.0) | 2<br>(0.6) |

The numerical value in a parenthesis indicates a percentage of the total number.

In Table 3, "IVS3+6113 $(GT)_n$" means a polymorphism in which, assuming that the base which is in intron 3 and in contact with exon 3 is 1, G and T located 6113 and 6114 bp downstream thereof are repeated n times. In addition, "IVS3+8761$(32bp)_n$" means a polymorphism in which one unit composed of 32 bp located 8761 bp downstream in the same manner in intron 3 is repeated n times.

The polymorphisms shown in Tables 1, 2 and 3 are included in the mu-opioid receptor gene polymorphisms of the present invention. Further, oligonucleotides containing the gene polymorphisms of the present invention are shown in Table 4. For example, the oligonucleotide of the present invention is an oligonucleotide selected from nucleotide sequences represented by SEQ ID NOS: 1 to 98 containing any of the above-mentioned polymorphisms and nucleotide sequences complementary to any of these (Table 4).

Table 4

| Position | Name of gene polymorphism | Sequence | SEQ ID NO |
|---|---|---|---|
| 5' UTR | A-5580G | gttcaactgctaataccttagcaggaatcgaaacagtgaccccatggcat a/g ctaagagtcactgtactcttcacagacgtgcactcacagaagaaaacac | 1 |
| | C-5342T | actaaagtagaatgcttgtcccaaagaaaagcgcatgttgcctgtttgag c/t tgtgaactaaattaaccactttttccgtggatcactattttttatttaaag | 2 |
| | A-5308G | atgttgcctgtttgagctgtgaactaaattaaccactttttccgtggatc a/g ctatttttatttaaagaatgactgaggccgggcgcggtggctcacgcctg | 3 |
| | C-5236T | ctgaggccgggcgcggtggctcacgcctgtaatcccagcactttgggagg c/t tgaggcaggcagatgacgaggtcaggagatcgagaccatcctggctaaca | 4 |
| | G-5026A | actcgggaggtggagcttgcagcgagctgagatcgcgccactgcactcca g/a cctgggcgacagagtgagactctgttttaaaataaataaataaataaaat | 5 |
| | G-4936A | ataaataaaataaaatataatgataaagaaatgtttttatagagctctca g/a ttttaatttctgaagtgatagactgtgataaagataacctaaataagaaa | 6 |
| | C-4504A | taattcttcttgctaatttctaggccacataccacaggatataaaaagcc c/a aacaacaaaggataaattctttcatatgtgtgtaatcctataaaccctct | 7 |
| | T-2694G | taaaatatatgctaatcatttttttcaactgaattcaaatattatgcacat t/g aatattcatatatgtttaatatagaaagaaacacagagagtgagggaggg | 8 |
| | A-2693C | aaaatatatgctaatcatttttttcaactgaattcaaatattatgcacatt a/c atattcatatatgtttaatatagaaagaaacacagagagtgagggaggga | 9 |
| | T-2683C | ctaatcatttttttcaactgaattcaaatattatgcacattaatattcata t/c atgtttaatatagaaagaaacacagagagtgagggagggagtccactatg | 10 |
| | T-2402C | aaaaatctatagtgttgtactgagctccctccaaagcaactataaattta t/c aggagatgaaacatatgattcaccaggcataagaagaaagtttccgtaat | 11 |
| | C-1609T | tccacatgaactaagcacaaaggaactgaatgcaggcagacagatttcag c/t tcaatataagagaattgttacattagttcatggaagaatatgttttaagg | 12 |
| | G-696T | tgtttctcatttctttttcagaaaataaaggatcgctgttgttcccaaca g/t gtttgtagggaagaaaattggagaaacattattaccttttcttagatgtt | 13 |
| | G-490T | tagggtttcatcaagccaatgtattccctgccagattttaaggagaaaaa g/t gcgctggaaaattgagtgatgttagccccctttcttatttttcactgcta | 14 |
| Exon 1 | A116G | ccccagcacccagccccggttcctgggtcaacttgtcccacttagatggc a/g acctgtccgacccatgcggtccgaaccgcaccgacctgggcgggagagac | 15 |
| Intron 1 | IVS1-A4980G | aatgaaaaggcagaaaaattagccccaaaagagatgaaactcttccgtcc a/g tcaccattgactctattgtgaacttatgaaaaaggtagttgagcaatatg | 16 |
| | IVS1-A4910G | gaacttatgaaaaaggtagttgagcaatatgaaggccatgatgtggaatt a/g aacacacacacacacacacacacacacacacacacacatgctggattctaaa | 17 |
| | IVS1-4908(AC)n | acttatgaaaaaggtagttgagcaatatgaaggccatgatgtggaattaa (ac)n atgctggattctaaaatgtgtccttcctcctctcactctcttgatcagtt | 18 |
| | IVS1-G4690A | acagaggtaatttatttagtctggcttcacttaacacaaataggtcaaaa g/a caatcacattttgtaagtagtaatagttggagaaatgtgtgaagaatagg | 19 |
| | IVS1-T4591G | ggtcaaaagataaataagaattattttataaccataagaaaggaagaaca t/g ctataaacaaaagtcatatatgcaacataaaagaataggtgagctgccag | 20 |
| | IVS1-T4456G | ttctggaagttccataaaaatcactctaatgggtcaaacatcgatggttc t/g cagaagaacacaattttttttcaaaaacgaatagcattgtaaattcatttg | 21 |
| Intron 2 | IVS2+G31A | tacaacaaaatacaggcaaggtgagtgatgttaccagcctgagggaagga g/a ggttcacagcctgatatgttggtgatgtcataagcaaagcagtatttatg | 22 |
| | IVS2+G518A | tttatatcaatatagacctcatggaggatctagctcatgttgagaggttc g/a tttttgttccctgaacgaaagcttaatgtgatcgaagtggactgcaaaat | 23 |
| | IVS2+G691C | ttccacaatttctttatagccttaagttagctctggtcaaggctaaaaat g/c aatgagcaaaatggcagtattaacacccttatgacataattaaatgttgct | 24 |
| Intron 3 | IVS3+G5807A | ctctaattactattattaaagcactttcttgacattttaatcaaaatagc g/a ggtcaagaagttaggagatgctctgtatttggtttaactgtgaactatat | 25 |
| | IVS3+G5953A | acatcactctcaaaagttgatctcagtttttttttacaagacatctgtgga g/a agttaatttgggaaagtaattgtttcaattcaatgggaaaaaaaactcaa | 26 |
| | IVS3+6113(GT)n | atcaaaatggctattctttcagttctacagtttaaaaagaaaatggttcc (gt)n gcgtgtgatataggcatgtctctttttgcatgtatggaattagagtaaat | 27 |
| | IVS3+A6151G | aaagaaaatggttccgtgtgtgtgtgtgtgtgtgtgtgcgtgtgatatag a/g catgtctctttttgcatgtatggaattagagtaaatgtaggtttaaaatt | 28 |
| | IVS3+A8449G | tgatatatatcataacatattatatattatattatgatatatatcataac a/g tgtattatcatattatgatatatatcataacatatatattatcatattac | 29 |
| | IVS3+C8497T | acatgtattatcatattatgatatatatcataacatatatattatcatat c/t acgatatatatcataacatattatatattatcatattatgatatatatca | 30 |
| | IVS3+8761(32bp)n | tatgacatatcataatatatattatcatattatgacatatcgtaatatat (attatcatattatgacatatatcataatatat) n atcaaaaagtca cagagctcatgcaagcccagtcatccccattgccagtg | 31 |
| | IVS3-G8804A | aatatatattatcatattatgacatatatcataatatatattatcatatt g/a tgacatatatcataatatatatcaaaaagtcacagagctcatgcaagccc | 32 |
| 3' UTR | TAA+G886A | taaaatgtactctttatttctcactggtttctccatactgcaggctcccc g/a catattattttctttttttaactcagctcagaatccttatgccttttgaa | 33 |
| | TAA+T1360C | atctaggtagacagccaagtcagatggcccatgcctagaagctctccatt t/c tgaacttttgtcagcattgattaaaagaatcaaataccttgtagttatct | 34 |
| | TAA+T1371C | cagccaagtcagatggcccatgcctagaagctctccattttgaacttttg t/c cagcattgattaaaagaatcaaataccttgtagttatctatgatgataca | 35 |
| | TAA+G1670A | ttatgtggactcaacccacgtatccagtagatgggaaaaaacaaaagcca g/a aataagtttttttagtgtttccttctgatgaagtttcatgtttgcttgtaa | 36 |
| | TAA+G1709A | aacaaaagccaaaataagtttttttagtgtttccttctgatgaagtttcat g/a tttgcttgtaataatctccatttctcaaatattatgttccataatagaca | 37 |
| | TAA+C2007T | atgcttttcatgggctaggatggtttctcccaagagatgacatagtattg c/t ttttgctcatcaggctgtttctcagcaatcattgtttctgcttaataccca | 38 |
| | TAA+A2109G | gctcctagtacgaattatctggcatgttgagagcaactttgtcttcaagt a/g ggacctgatctatctttttccacaaatgtcatgtgtgtgaacaagtttct | 39 |
| | TAA+A2274G | attctaaagtaaataataaataaggtcattgtcaacgtttttcattcaaa a/g ccatttttttaacgtaaatttgctagaaccaccttccaattccaaggcaag | 40 |

Table 4-2

| Position | Name of gene polymorphism | Sequence | SEQ ID NO |
|---|---|---|---|
| | TAA+G2287A | taataaataaggtcattgtcaacgtttttcattcaaaaccattttttaac g/a taaatttgctagaaccaccttccaattccaaggcaaggagagacattaca | 41 |
| | TAA+G2395C | ctcaactggatgggctaaggtttctgataaaatctgaagataaagaaaat g/c gaatattctgctttttttcttccttctaatttcacccttgcctaaggatga | 42 |
| | TAA+G2458C | tttttcttcattctaatttcacccttgcctaaggatgagatttcttccca g/c gttggtatcccagaaatgcagactgtagctatggggcggaagctttgttt | 43 |
| | TAA+T2482C | ttgcctaaggatgagatttcttcccaggttggtatcccagaaatgcagac t/c gtagctatggggcggaagctttgtttctttacctgatcacttgctgtgga | 44 |
| | TAA+G2497A | atttcttcccaggttggtatcccagaaatgcagactgtagctatggggcg g/a aagctttgtttctttacctgatcacttgctgtggaaattctagcttattg | 45 |
| | TAA+G2656T | tccctctttccttgccaatcattagaaaggaaagaagaggaaagagactc g/t ctggagcactggtgagtctctaggaccctgctatcctatcccaacagggc | 46 |
| | TAA+C2714A | actggtgagtctctaggaccctgctatcctatcccaacagggctgtcaga c/a ggagaactcctaatgtggccatttgaaacacttctcaacattgaaataga | 47 |
| | TAA+G2820T | gaagttttaaaataacctcttctaagacacggctatgagtaggtaagaga g/t cattcattcccttcaataatatgactgtgttgataaaactgataaccatt | 48 |
| | TAA+G2907T | aactgataaccattcacttgcaaatgttattattgaataagtctcactta g/t ctcatttaatattaccccaaagatgctaacaaattctgtttcccacattg | 49 |
| | TAA+T3423C | gccaaagcaacctaagaataggacatggtagcttaagtttttcagcttct t/c aactggccacacacacacaagttgtgtttgtacaattcttgaggtcaatc | 50 |
| | TAA+A4026G | caaacaatattactgtgttctaagcgcttctgttactcgaaaggggtctg a/g tccagaccccaaaagagggttcttggacctcatgcaagaaagaattcagg | 51 |
| | TAA+4585(A)n | ggtttgttttaagtaagccactttcctccctgcaagttcccacggagcag (a)n ggaggaaacttttttcctgggagcccactaatcacacagtgaacaaaaggc | 52 |
| | TAA+A4861C | taagaaagcaaaggaataaagaatggctactccataggcagcgtagcccc a/c agggctgctggttggctatttttgtggttatttcttgattatatgctaaa | 53 |
| | TAA+A5359G | gtcgctctggttcaaacacctctgacacttgaattacaaatataaggacc a/g ttgacactgagatttttaagggaggaaaaacagattgacagtggactaaag | 54 |
| | TAA+A6074C | gcaaggtaagaatcaagtagaaatgataaagggcaaggaaaaaagatgaa a/c gcttactcatattaaccattctaccattggaattatttgccaacacacct | 55 |
| | TAA+T6866G | gacagtggggaaaattcatcttcatattgtcacatgcactgtaataggaa t/g gtttagcaaaaaaaaccttccagagaaaggtggtttccaatattacctac | 56 |
| | TAA+C6922G | gcaaaaaaaaccttccagagaaaggtggtttccaatattacctacaactt c/g ctttgcaatttgatttttgaaaggacctaaaagttgaaaacaggctatca | 57 |
| | TAA+7075del(322bp) | taaatgttttatttaagtttgcattgcccactaaggctagacatttttttt del(322bp*1) gataaattcacagggttacaaaataccaaacggaaatgagataagtggta | 58 |
| | TAA+C7427T | ggcccggctagacatttttttgataaattcacagggttacaaaataccaaa c/t ggaaatgagataagtggtataaaccacagaagatatataggagaagagaaaa | 59 |
| | TAA+7483del(A) | tgagataagtggtataaaccacagaagatatataggagaagagaaaaaaaaa del(a) gaggaaataaagaagacaactcttttcctaagagtctgggtaaaattgaa | 60 |
| | TAA+T7536C | ggaaataaagaagacaactcttttcctaagagtctgggtaaaattgaaca t/c agccatattcactgaacaacatgagtgagcttcattaatttaagcacagc | 61 |
| | TAA+A7589G | ccatattcactgaacaacatgagtgagcttcattaatttaagcacagcaa a/g actgctttaattaacaagaccagagagaagggagaggagactacatttgt | 62 |
| | TAA+C8116T | gtgacatattagacttcttactttccccaaataaaaaagtgcctgctggg c/t gcggtggctcacgcctgtaattccagcactttgggaggccgaggcgggcg | 63 |
| | TAA+C8165T | gcgcggtggctcacgcctgtaattccagcactttgggaggccgaggcggg c/t ggaacacaaggtcaggagatcaagaccatcctggccaatatggtaaaacc | 64 |
| | TAA+G8281A | atacaaaattaggaaggcgtggtggtgcacgcctgtaatcccagctagtc g/a ggaggctgaggcaggagaattgcttgaactggggaggcggaagttgcagt | 65 |
| | TAA+8386(A)n | caagatcgcagcattgcactccagcctgggcaacagaatgagattgtctc (a)n gtgccacatgccatgctatgtgcccaaagtttccttcacacaacacagcc | 66 |
| | TAA+C9000T | ttagagccagtcagaattcaatctccaatatcctgactagcacaagaaat c/t cataggttgattcttgttctcctgcatctctgcaggtggcaaacctgatt | 67 |
| | TAA+A9564G | ttgtgtgttttcttaataaactttacccacttattaaaagaataaaatga a/g ggtggagttaattctgactacgggattcctttttcacttttataatgaac | 68 |
| | TAA+G9569A | tccttctaactaaatcttatcataagcaaatctatgcaccaaattattta g/a tacaattcctaataacagctgaaggaccatttatttgaagcaatgttcac | 69 |
| | TAA+T9716A | ttagtacaattcctaataacagctgaaggaccatttatttgaagcaatgt t/a caccatagcaaaattccagtgaagtctaagaactgggacagtccgttgag | 70 |
| | TAA+T9839G | ttgccccatgaatgtgcacatgcatattaaaatatgggcacctcttttaa t/g tctttttttttctcataataagtttgaaactcacagtaggaaattgagaga | 71 |
| | TAA+C9994A | tggttgttctcgaactagctggtttcccagagacagctggagactgagca c/a ataaagacatcattgaggaaaaaggctaccttgtacctcatggagagctg | 72 |
| | TAA+C10083A | catggagagctgaaggtctgataaatgggaactgccaggtaatagctatg c/a tatttctgacataaatttaaaaactagtattgtttcttctagctctgttt | 73 |
| | TAA+10223(A)n | taatgttaaattggatctataaacataagtcaatttggctctattatgtc (a)n gagaataggagttttaacttatatctgtgttttattaatattttgaagta | 74 |
| | TAA+A10247T | ataagtcaatttggctctattatgtcaaaagagaataggagttttaactt a/t tatctgtgttttattaatattttgaagtataggaacctcatggtgtagca | 75 |
| | TAA+A10535G | gtatgtgacagggctgcatgcaccggtggtctgggaggaacagaacagg a/g cagggagttcttctatacaatagagaacagaacaatgttcttctatacaa | 76 |
| | TAA+G10704A | aggcgggcccaggcctggtttcgggcctggcgctgagctgcctgtatttg g/a ttttacttccttgttgtttttactgaatatgaaacaatataaaacaatgt | 77 |
| | TAA+T10752G | tggttttacttccttgttgtttttactgaatatgaaacaatataaaacaa t/g gtgagagggtctttctctcctctcaatgtcaacatcatatatgattggag | 78 |
| | TAA+C11100T | gctggtttggttgaagtttctcttatcagtcaggcactttgcattttaag c/t gtactttaccaccgacaccctcccccccagcacacacacacacacacac | 79 |
| | TAA+C11129A | tcaggcactttgcattttaagcgtactttaccaccgacaccctccccccc c/a agcacacacacacacacacacacacacacacacacaacatagtgaaatgg | 80 |

Table 4-3

| Position | Name of gene polymorphism | Sequence | SEQ ID NO |
|---|---|---|---|
| | TAA+11132(CA)n | ggcactttgcattttaagcgtactttaccaccgacaccctcccccccag (ca)$_n$ acatagtgaaatggacccgtgggaattatatgatagttgtaatcaaaata | 81 |
| | TAA+A11133G | gcactttgcattttaagcgtactttaccaccgacaccctcccccccagc a/g cacacacacacacacacacacacacacacacaacatagtgaaatggaccc | 82 |
| | TAA+11368del(TCTC) | tctggaagtaaacttaaaatgaaaattagaatttgctttcaattatacta del(tctc) tatctaaatcttaatttgaaatttaaattattttgtctctacccaaacca | 83 |
| | TAA+T11411C | tatactatctctatctaaatcttaatttgaaatttaaattattttgtctc t/c acccaaaccatcgatttcatggaaatgtttaaattttctttttttttttt | 84 |
| | TAA+T11431C | cttaatttgaaatttaaattattttgtctctacccaaaccatcgatttca t/c ggaaatgtttaaattttctttttttttttttttttgatggagtctcact | 85 |
| | TAA+11449ins(TTTC) | tattttgtctctacccaaaccatcgatttcatggaaatgtttaaattttc ins(tttc) tttttttttttttttttgatggagtctcactctgtcgcccaggctggag | 86 |
| | TAA+G11541A | aggctggagtgcagtggctcaatcttggctcactgcaacctctgcctccc g/a ggttcacaccattctcctgcttcagcctcctgagtagctgggactacagg | 87 |
| | TAA+A11602C | attctcctgcttcagcctcctgagtagctgggactacaggtgcccgccac a/c acacctggctaatttttgtatttttagtagagatggggtttcaccacgt | 88 |
| | TAA+C11650T | acaacacctggctaatttttgtatttttagtagagatggggtttcacca c/t gttagccaggatggtttcgatctcctgacctcgtgatctgcctgcctcgg | 89 |
| | TAA+C11918T | atccccatcaatttaataggaattaagttagaaatactagtatatatatt c/t cctttatatactaattgtatatccatataaaagcattagtaccattatat | 90 |
| | TAA+A11956C | agtatatatattccctttatatactaattgtatatccatataaaagcatt a/c gtaccattatatgaaagtatatatgccattccataaaatatatctacca | 91 |
| | TAA+A12143G | ggaattaaagaaaaaatgcctgttttcactaagtcatccttcccctggca a/g tacatttcctgaacttttacatacttaaatagccagttatgaaaatgtaa | 92 |
| | TAA+A12630G | acattttaaacagactcctgcccacaaactatttttcctctccaggaata a/g gaatggcaactgaattgttcctctttattctatagctttaagtcaaacc | 93 |
| | TAA+T12681C | gaatggcaactgaattgttcctctttattctatagctttaagtcaaacc t/c aacataagcaatcaacccttccacccattgtcctctttctagctgcttat | 94 |
| | TAA+T12831C | ttgggggtgaaataaaagatagacccctgctgctctgcacgtagattcag t/c ttgtatgccagggtgacattttaatttacagtagtccagacacctaaaca | 95 |
| | TAA+G12834C | ggggtgaaataaaagatagacccctgctgctctgcacgtagattcagttt g/c tatgccagggtgacattttaatttacagtagtccagacacctaaacagga | 96 |
| | TAA+13236(T)n | caacattgttttcctttgatggtctgggagtttttctataagtttttgg (t)$_n$ ctcttcattagtgtgttagttccatcatcatgtctgtttactattgaaaa | 97 |
| | TAA+T13971G | ttgaaaatataggcagctaaatccactgatagtctactttttttaaaaat t/g tgttcttgatgttttgagcaggaaaattatttgcaagaaacaaagagttt | 98 |

*1 322 bp deletion
tttttttttttttttttttttttttgagacagagtctcgctctgtcgcccaggctggagtacagtggcgggatctcggctc
actgcaagctccgcctcccgggttcacgccattctcctgcctcagcctcccaagtagctgggactacaggcgcccgccac
tacgcccggctaatttttgtatttttagtagagacggggtttcaccgttttagccgggatgatctcgatctcctgacct
cgtgatccgcccgcctcggcctcccaaagtgctgggattacaggcgtgagccaccgcgcccggcccggctagacattttt

In Table 4 (SEQ ID NOS: 1 to 98), 101 bases are shown, and a polymorphic site is shown at the 51st base. One represented by "a/g" means a polymorphism associated with "a" and "g", and "c/t" means a polymorphism associated with "c" and "t". Further, "$(ac)_n$" means a polymorphism in which "ac" is repeated n times, "del (a)" means a deletion polymorphism of "a", and "ins(tttc)" means an insertion polymorphism of "tttc". Other bases are indicated in the same manner. Among these, in the present invention, an oligonucleotide having at least 10 bases, preferably 10 to 100 bases, more preferably 10 to 45 bases, further more preferably 14 to 25 bases containing a polymorphic site (the 51st base) in any of the nucleotide sequences (SEQ ID NOS: 1 to 98) shown in Table 4 and an oligonucleotide complementary to this are provided. Here, the "51st base" means a base at a polymorphic site, and the base is not necessarily one base. Accordingly, the base is not present in a deletion polymorphism, therefore, the base is decided not to be counted in the number of bases, and even if a plurality of bases are repeated, they are decided to be counted as the "51st base".

Incidentally, in Table 4, a sequence of 322 bp contained in a gene polymorphism represented by "TAA+7075dsl (322bp)" (SEQ ID NO: 58) is shown below and outside the table.

3. Haplotype analysis

In the present invention, by using SNP among the above-mentioned gene polymorphisms, a haplotype can be constructed. The SNP to become a target of a haplotype analysis may be any as long as its polymorphism frequency is 0.5% or higher, preferably, those with a polymorphism frequency of 1%, more preferably those with a polymorphism frequency of 5% or higher can be selected. Further, SNP to become a target of a haplotype analysis may be a full or partial sequence thereof.

The haplotype analysis can be carried out using various computer programs, and for example, Arlequin program (available from http://anthro.unige.ch/arlequin) (Schneider S, Roessli D, Excoffier L. Arlequin 2000: a software for population genetics data analysis. Ver 2.000. Genetics and Biometry Lab, Dept of Anthropology Univ of Geneva.) can be used.

As an example of the haplotype analysis, as for the 10 sites of SNPs with a polymorphism frequency of 5% or higher among polymorphisms found as in the above-mentioned 2, a haplotype is estimated using Arlequin program. At this time, as for the groups of SNPs showing complete linkage disequilibrium present in a region from intron 3 to the 3' untranslated region, one of them, for example, "TAA+A2109G" can be used as a representative. The estimated haplotypes are shown in Table 5.

Table 5: Haplotype of mu-opioid receptor gene polymorphism in normal subjects

| No. | frequency | A118G | IVS2 +G691C | IVS3+G5807A | IVS3 +G5953A | IVS3 +A6151G | IVS3 +A8449G | IVS3 +C8497T | TAA +A2109G | TAA +A2274G | TAA +G2287A |
|---|---|---|---|---|---|---|---|---|---|---|---|
| 1 | 4.9% | A | C | A | G | G | A | C | A | A | A |
| 2 | 3.0% | A | C | G | G | G | A | C | A | A | A |
| 3 | 24.6% | A | C | G | G | G | A | T | A | A | A |
| 4 | 6.5% | A | G | G | A | G | A | C | A | G | A |
| 5 | 5.4% | A | G | G | G | A | G | C | G | A | G |
| 6 | 35.9% | G | C | A | G | G | A | C | A | A | A |
| 7 | 3.6% | G | C | G | G | G | A | T | A | A | A |
| 8 | 18.1% | 27 haplotypes with lower frequencies less than three percents | | | | | | | | | |
| sum | 100.0% | | | | | | | | | | |

As for the haplotypes of the mu-opioid receptor gene polymorphism in normal subjects, 34 haplotypes are estimated, and among these, there are 7 haplotypes observed at a high frequency of 3%, and 27 haplotypes which is estimated to occur at a frequency of 3% or lower are collectively shown in No. 8 of Table 5.

Subsequently, based on the above-mentioned haplotype frequency, a linkage disequilibrium analysis is carried out. The D' value and $r^2$ value, which indicate measures of linkage disequilibrium, can be calculated based on the following definition.

Definition:

Assuming that there are SNP A and SNP B, and the respective alleles are represented by A and a, and B and b. The four haplotypes formed by SNP A and SNP B are represented by AB, Ab, aB and ab, and the respective haplotype frequencies are represented by $P_{AB}$, $P_{Ab}$, $P_{aB}$ and $P_{ab}$.

$$D = P_{AB} \times P_{ab} - P_{Ab} \times P_{aB}$$

(In the case of D > 0)

$$D' = (P_{AB} \times P_{ab} - P_{Ab} \times P_{aB})/\mathrm{Minimum}\ (((P_{AB} + P_{aB}) \times (P_{aB} + P_{ab})), ((P_{AB} + P_{Ab}) \times (P_{Ab} + P_{ab})))$$

(In the case of D < 0)

$$D' = (P_{AB} \times P_{ab} - P_{Ab} \times P_{aB})/\mathrm{Minimum}\ (((P_{AB} + P_{aB}) \times (P_{AB} + P_{Ab})), ((P_{aB} + P_{ab}) \times (P_{Ab} + P_{ab})))$$

$$r^2 = (P_{AB} \times P_{ab} - P_{Ab} \times P_{aB})^2/\{(P_{AB} + P_{Ab})(P_{AB} + P_{aB})(P_{aB} + P_{ab})(P_{Ab} + P_{ab})\}$$

[However, Minimum ((($P_{AB}$ + $P_{aB}$) x ($P_{aB}$ + $P_{ab}$)), (($P_{AB}$ + $P_{Ab}$) x ($P_{Ab}$ + $P_{ab}$))) means that a smaller value among ($P_{AB}$ + $P_{aB}$) x ($P_{aB}$ + $P_{ab}$)] and (($P_{AB}$ + $P_{Ab}$) x ($P_{Ab}$ + $P_{ab}$)) is adopted.]

Table 6 shows one example showing linkage disequilibrium among mu-opioid receptor gene polymorphisms in normal subjects

Table 6: Linkage disequilibrium among mu-opioid receptor gene polymorphisms in normal subjects

D'

| Locus | A118G | IVS2 +G691C | IVS3 +G5807A | IVS3 +G5953A | IVS3 +A6151G | IVS3 +A8449G | IVS3 +C8497T | TAA +A2109G | TAA +A2274G | TAA +G2287A |
|---|---|---|---|---|---|---|---|---|---|---|
| A118G | | 0.795 | 0.704 | 0.626 | 0.866 | 0.884 | 0.633 | 0.875 | 0.458 | 0.890 |
| IVS2 +G691C | 0.125 | | 1.000 | 0.714 | 0.746 | 0.795 | 0.775 | 0.772 | 0.672 | 0.795 |
| IVS3 +G5807A | 0.453 | 0.134 | | 0.921 | 0.849 | 0.883 | 0.895 | 0.868 | 0.535 | 0.883 |
| IVS3 +G5953A | 0.052 | 0.341 | 0.102 | | 0.023 | 0.009 | 1.000 | 0.034 | 0.742 | 0.009 |
| IVS3 +A6151G | 0.054 | 0.201 | 0.047 | 0.000 | | 1.000 | 1.000 | ***1.000*** | 1.000 | ***1.000*** |
| IVS3 +A8449G | 0.069 | 0.284 | 0.063 | 0.000 | 0.001 | | 1.000 | ***1.000*** | 1.000 | ***0.967*** |
| IVS3 +C8497T | 0.159 | 0.062 | 0.291 | 0.069 | 0.037 | 0.046 | | 1.000 | 0.763 | 1.000 |
| TAA +A2109G | 0.061 | 0.241 | 0.055 | 0.001 | ***0.892*** | ***0.901*** | 0.042 | | 1.000 | ***1.000*** |
| TAA +A2274G | 0.024 | 0.266 | 0.030 | 0.484 | 0.011 | 0.014 | 0.035 | 0.014 | | 1.000 |
| TAA +G2287A | 0.070 | 0.284 | 0.063 | 0.000. | ***0.800*** | ***0.934*** | 0.046 | ***0.989*** | 0.014 | |

In the results of the linkage disequilibrium analysis, a combination with a D' value (upper right of the table) being significant and 0.7 or higher is underlined, and a combination with an $r^2$ value (lower left of the table) being significant and 0.5 or higher is represented by a bold and italic form.

In Table 6, when the loci are named beginning at the top such that A118G is named "SNP1", IVS+G691C is named "SNP2", and so on, and TAA+G2287A is named "SNP10", the linkage disequilibrium between SNP1 and SNP2 is calculated and the resulting value is written in the cell at the intersection of the row of SNP1 and the column of SNP2 (upper right of the table). The value of D', which is an index of the linkage disequilibrium, of 0.7 or higher is underlined. Further, an $r^2$ value, which is a more stringent index of the linkage disequilibrium, is calculated in the same manner, and the resulting value is written in the cell at the intersection of the row of SNP1 and the column of SNP2 (lower right of the table).

When attention is given to D' in Table 6, except for IVS3+G5953A and TAA+A2274G, significant linkage disequilibrium has been observed in all the combinations of SNPs. Further, when attention is given to $r^2$ values, significant linkage disequilibrium can be observed in three SNPs in intron 3 and the 3' untranslated region, and one of them is TAA+A2109G.

Further, in the present invention, the linkage disequilibrium between a repeat polymorphism and each SNP can also be analyzed. For example, the repeat polymorphisms shown in Table 3 exhibit diversity, however, significant linkage disequilibrium between them and all the 10 SNPs shown in Table 6 could be observed ($P < 0.05$).

Further, in the present invention, from the results of the linkage disequilibrium analysis (Table 6), four haplotype blocks can be estimated. As for the haplotype block, from the results of the haplotype analysis, for example by using HaploBlockFinder Version 0.6, a linkage block can be estimated (http://cgi.uc.edu/cgi-bin/ kzhang/haploBlockFinder.cgi).

From this estimation, the SNPs shown in Table 6 can be classified into 4 haplotype blocks, i.e., a block containing A118G, a block containing IVS2+G691C and IVS3+G5807A, a block containing IVS3+G5953A and a block composed of SNP downstream of IVS3+A6151G.

When a specific SNP in the estimated haplotype blocks is examined, the information of SNPs indirectly linked to each other in the same block can be obtained. That is, when a gene polymorphism of the mu-opioid receptor is examined, it is not necessary to analyze all the SNPs, and it is only necessary to perform typing for several specific SNPs.

4. Correlation between mu-opioid receptor gene polymorphism and drug sensitivity

It is considered that when a polymorphism occurs in the mu-opioid receptor gene, the expression level or function of mu-opioid receptor changes. Therefore, there is a correlation between a mu-opioid receptor gene polymorphism and various phenotypes associated with the mu-opioid receptor in some cases. Here, as the phenotype, a phenotype associated with occurrence of a disease and a phenotype associated with drug sensitivity can be exemplified. As the phenotype associated with occurrence of a disease, pain sensitivity, vulnerability to drug addiction and the like can be exemplified. As the phenotype associated with drug sensitivity, an efficacy of a drug, a side effect of a drug, duration of efficacy of a drug and the like can be exemplified.

A correlation between a mu-opioid receptor gene polymorphism and a phenotype can be examined as follows. As for the mu-opioid receptor gene polymorphism, as a result of the linkage disequilibrium analysis and the haplotype analysis in normal subjects, a representative polymorphism in the estimated linkage blocks, for example SNP is selected. Then, a polymorphism frequency is analyzed for this polymorphism (such as SNP) in individual test subjects (patients), and comparison thereof with a polymorphism frequency of normal subjects is carried out. In the comparison, it is effective that a statistical technique such as a chi-square test is used.

In the case where the phenotype or the symptom is a stimulant-induced psychotic-like symptom, it can be classified, for example, according to a period of time from the start of the use of a stimulant to the occurrence of delusion or hallucination, a period of duration of delusion or hallucination after termination of the use thereof, the presence or absence of the relapse, and the presence or absence of multiple drug abuse. Then, for each class, the polymorphism frequencies or genotypes of normal subjects and test subjects are compared. As a result, a polymorphism with a significant difference in the polymorphism frequency compared with the control group can be used for evaluating the susceptibility to a disease or the difference in drug sensitivity. However, it is suggested that the tendency of gene polymorphism is affected by the race, birthplace or the like, it is preferred that in a group showing a similar gene polymorphism to that of a population used for finding an associated polymorphism (such as SNP), the above-mentioned evaluation using the polymorphism is carried out.

5. Use of analysis results

The results analyzed as described above can be used as an index in a method of predicting sensitivity to various drugs associated with the mu-opioid receptor, a method of selecting a method of treating or preventing a disease associated with the mu-opioid receptor, a method of determining an appropriate administration amount of a therapeutic drug, a method of predicting side effects or the like. Incidentally, by determining the genotype of an individual with the use of the oligonucleotide or the microarray of the present invention, it becomes possible to perform the prospective evaluation of drug sensitivity.

As the drug associate with the mu-opioid receptor (function modulator), a stimulant such as methamphetamine, a dopamine receptor agonist, a dopamine receptor antagonist, a μ-, κ-, or δ-opioid receptor agonist, a μ-, κ-, or δ-opioid receptor antagonist, alcohol and the like can be exemplified, and examples thereof may include methamphetamine methylenedioxymethamphetamine, amphetamine, dextroamphetamine, dopamine, morphine, DAMGO, codeine, methadone, carfentanil, fentanyl, heroin, cocaine, naloxone, naltrexone, nalorphine, levallorphan, pentazocine, buprenorphine, oxycodone, hydrocodone, levorphanol, etorphine, dihydroetorphine, hydromorphone, oxymorphone, ethanol, methanol, diethyl ether, tramadol and the like.

Further, by using the gene polymorphism or the method of the present invention, it is possible to evaluate drug sensitivity or the like in different races. The subjects are not particularly limited and examples thereof include Japanese, Europeans, Americans and the like, however, in the present invention, they are preferably Japanese or those having a similar gene polymorphism tendency to that of Japanese.

Morphine is used as an analgesic drug, however, it also has an effect on suppressing respiration, therefore it is necessary to determine its administration amount carefully. If a correlation between the novel gene polymorphism which was elucidated by the present invention and the sensitivity to morphine has been analyzed and each individual has been determined in advance for what polymorphism confers what sensitivity to morphine in it, by examining a mu-opioid receptor gene polymorphism of a patient to whom morphine is to be administered, the sensitivity to morphine of the patient is elucidated and an administration amount can be determined.

A genome sample of a test subject can be extracted from the blood, saliva, skin or the like, however, the origin is not limited to these as long as a genome sample can be collected therefrom. The extraction and purification methods of genomic DNA is publicly well known. For example, genomic DNA is purified from a specimen such as the blood, saliva, skin or the like collected from a human using the phenol method or the like. At this time, a commercially available genomic DNA extraction kit such as GFX Genomic Blood DNA Purification Kit or a device may be used. In the case where SNP to be examined is in an open reading frame, mRNA or total RNA may be extracted instead of genomic DNA. Hereinafter, an example of the gene polymorphism detection method used for the above-mentioned test sample will be shown.

(1) Detection using the PCR method

In order to amplify a test sample by PCR, it is preferred that a high fidelity DNA polymerase, for example, KOD Dash polymerase (TOYOBO) is used. A primer to be used is designed such that a gene polymorphism is contained at a given position of the primer so as to amplify a target SNP in the test sample and synthesis is carried out.
After completion of the amplification reaction, detection of the amplified products is carried out, and the presence or absence of a polymorphism is determined.

(2) Detection by the sequencing method

In the present invention, the polymorphism of the present invention can also be detected by a sequencing method based on the dideoxy method. As a sequencer to be used for the sequencing, a commercially available ABI series (Amersham Biosciences) is used.

(3) Detection by a DNA microarray

A DNA microarray is a microarray in which a nucleotide probe has been immobilized on a support, and includes a DNA chip, a Gene chip, a microchip, a bead array and the like.
First, a polynucleotide of a test sample is isolated and amplified by PCR, and then labeled with a fluorescent reporter group. Then, a labeled DNA/mRNA, total RNA are incubated along with an array. Then, this array is inserted in a scanner, and a hybridization pattern is detected. The data of the hybridization is collected as emitted light from the fluorescent reporter group bound to the probe array (i.e., incorporated in a target sequence). A probe which is completely identical with the target sequence generates a stronger signal than those having a region which is not identical with the target sequence. Because the sequence and the position of each probe on the array are known, the sequence of the target polynucleotide reacted with the probe array can be determined based on the complementarity.

(4) Detection by the TaqMan PCR method

The TaqMan PCR method is a method utilizing an allele specific oligonucleotide labeled with fluorescence and a PCR reaction with Taq DNA polymerase. The allele specific oligonucleotide (referred to as TaqMan probe) to be used in the TaqMan PCR method can be designed based on the above-mentioned gene polymorphism information.

(5) Detection of gene polymorphism by the invader method

The invader method is a method of detecting a gene polymorphism by subjecting an allele specific oligonucleotide and a template to hybridization. A kit for carrying out the invader method is commercially available, and it is possible to easily detect a gene polymorphism by this method.

6. Kit

In the present invention, an oligonucleotide containing a mu-opioid receptor gene polymorphism (such as SNP) can be included in a kit for detecting a gene polymorphism.
The kit for detecting a gene polymorphism of the present invention includes one or more components necessary for carrying out the present invention. For example, the kit of the present invention includes a component for storing or supplying an enzyme and/or a reaction component necessary for detecting a gene polymorphism. Such a component is not limited, however, examples thereof include the oligonucleotide of the present invention, an enzyme buffer solution, dNTP, a reagent for control (such as a tissue sample or a target oligonucleitide for a positive or negative control), a reagent for labeling and/or detection, a solid phase support, a written instruction manual and the like. Further, the kit of the present invention may be a partial kit including a part of the necessary components. In this case, a user can prepare the other components.
The kit of the present invention can be provided as a microarray in which the above-mentioned oligonucleotide has been immobilized on a support. The microarray is one in which the oligonucleotide of the present invention has been immobilized on a support, and includes a DNA chip, a Gene chip, a microchip, a bead array and the like.
The kit of the present invention includes an oligonucleotide containing a gene polymorphism found in the present invention. Therefore, the blood is collected before a drug is applied to a patient or the like (for example, before surgery, at the time of occurrence of cancer pain or the like), and a mu-opioid receptor gene is isolated. Then, this gene is reacted with an oligonucleotide in the kit, whereby a genotype is determined. From the determined genotype, a dosage regimen such as the type or dose of the drug is formed. As a result, an effect of the drug suitable for an individual can be obtained, which is useful in the personalized medicine. For example, in the case of using morphine, it becomes possible to obtain an analgesic effect suitable for an individual, and also to suppress the side effects to the minimum.

Hereinafter, the present invention will be described in more detail with reference to Examples, however, the invention is not limited to Examples.

[Example 1] SNP analysis and haplotype construction

(1) Subjects

The present Example was carried out by using as subjects, 128 methamphetamine-addicted patients (99 males, 29 females, the average age of 35.9 ± 1.0) who show F15.2 and F15.5 based on the diagnostic criteria ICD-10 DCR of WHO and were hospitalized in or had been released from a psychiatric hospital or department of psychiatry in Japan belonging to a study group of analyzing genome associated with drug addiction (Japanese Genetics Initiative for Drug Abuse (JGIDA)), and 179 normal subjects (139 males, 40 females, the average age of 34.6 ± 1.5) who were mainly engaged in a medical service and did not have a history of drug addiction in the past or did not have any family member who had a history of drug addiction, and did not suffer from a mental disease.

Further, as a result of analysis of polymorphism frequency in normal subjects, SNP was observed with a frequency which was significantly different from the polymorphism frequency reported so far in exon 1 and intron 2, therefore, as for these two regions, 34 and 53 normal subject samples were added, respectively and an analysis was carried out.

The diagnosis was carried out based on an interview by two psychiatrists and all the available information including the records of the hospital. Further, patients with a history of schizophrenia, organic psychosis syndrome, or another mental disease were excluded from the addicted patients.

The test subjects were all Japanese, and their birthplaces and places of residence were Kitakyusyu, Setouchi, Tokai and Kanto regions.

The present Example was carried out after the approval by ethical review in each institute belonging to JGIDA was obtained and a written consensus on the use of the genome sample in this study was obtained by all the test subjects.

(2) Analysis for cases classified according to stimulant-induced psychotic-like symptoms

In an analysis performed after classifying the methamphetamine-addicted patients based on the clinical information, the patients were classified into groups in terms of the following 4 items.

(A) Latency of psychosis (the period of time from the start of the use to the occurrence of delusion or hallucination)

- Occurred wi thin 3 years (n = 54, the average number of years = 0.83 year)
- Occurred after a lapse of not less than 3 years (n = 53, the average number of years = 9.98 years)

(B) Prognosis of psychosis (the period of duration of delusion or hallucination after termination of the use)

- transient (n = 72, disappeared within one month)
- middle (n = 17, disappeared within a period of one month to 6 months)
- prolong (n = 26, continued for not less than 6 months)

(C) Spontaneous relapse (the presence (n = 86) and the absence (n = 42) of relapse)
(D) Poly drug abuse (multiple drug abuse)

- none (n = 36, single abuse of methamphetamine)
- easily accessible drug only (n = 49, with a history of abusing alcohol or thinner)
- heavy (n = 43, with a history of abusing morphine, heroin, cannabis or the like)

Further, the methamphetamine-addicted patients for whom the clinical information in terms of the respective items could not be obtained were excluded from this analysis.

(3) Genotyping

A genome sample was purified from a blood specimen collected from each test subject using a standard phenol extraction method. More specifically, to the whole blood, 3-fold volume of a hemolysis reagent (0.1 mM EDTA, 155 mM

$NH_4Cl_2$) was added, and the mixture was left at room temperature for 10 minutes and then centrifuged. Then, the precipitate was resuspended. The resulting suspension was subjected to a proteinase K treatment and an RNase treatment and then centrifugation. Then, from the resulting supernatant, phenol/chloroform extraction was carried out, followed by ethanol precipitation, whereby genomic DNA was prepared. By using the genomic DNA as a template, the PCR method was carried out using KOD Dash (TOYOBO, Tokyo), and the resulting product was purified by ethanol precipitation, then a template DNA for sequencing was prepared.

As for a 5' side untranslated region (5' UTR) and 5' flanking region of a mu-opioid receptor gene, a full region of interest, that is, a region up to 5765 bp upstream from the start codon was amplified by carrying out PCR amplification of two regions (-5765 to -2272 and -2612 to 421). As for exon 1, exons 2 to 3 (containing intron 2), exon 4 (containing a region 2952 bp downstream from the stop codon) and a part of introns 1 and 3, amplification of DNA fragment was carried out by the PCR method for each region (see Fig. 1 for the amplified regions). As for a 3' side untranslated region (3' UTR and 3' flanking region), a full region of interest, that is, a region up to 13830 bp downstream from the stop codon was amplified by carrying out PCR amplification of four regions (TAA+2004 to 6288, 4980 to 10376, 8292 to 12120, and 11260 to 14677). The base sequence was decoded by the sequencing method with the use of Big Dye Terminator V3.1 Cycle Sequencing Ready Reaction (Applied Biosystems, Tokyo) by using these PCR products as templates and an analysis of gene polymorphisms was carried out.

First, by using 44 cases of the genome samples of normal subjects, for the full region of the amplified PCR fragments, a polymorphism observed at a high frequency on a mu-opioid receptor gene was found with the use of the 152 primers in total. Then, with regard to the entire exon region and a region containing a polymorphism observed at a high frequency (allelic frequency > 5%), by using the genomic DNA of the remaining normal subject samples and methamphetamine-addicted patient samples, an analysis of mu-opioid receptor gene polymorphism was carried out. The primers were designed with reference to gene sequences published in National Center of Biotechnology Information (GenBank Accesion No. NT-023451).

(4) Statistic analysis

A significant difference test for polymorphism frequency between normal subject samples and methamphetamine-addicted patient samples was carried out using a chi-square test (significance level $\alpha = 0.05$). Further, in order to confirm the bias of samples for all the polymorphisms, examination of Hardy-Weinberg (HW) equilibrium was carried out using a chi-square test. As for a linkage disequilibrium analysis and a haplotype analysis, the genotype information of 179 cases of normal subjects and 128 cases of methamphetamine-addicted patients was analyzed using Arlequin program (available from http://anthro.unige.ch/arlequin) (Schneider S, Roessli D, Excoffier L. Arlequin 2000: a software for population genetics data analysis. Ver 2.000. Genetics and Biometry Lab, Dept of Anthropology Univ of Geneva). Then, a D' value and $r^2$ value, which indicate the measures of linkage disequilibrium, were calculated based on the definition described above.

The significant difference of polymorphism frequency between normal subject samples and methamphetamine-addicted patient samples in a repeat polymorphism was obtained using CLUMP program produced based on the Monte Carlo method (Sham PC, Curtis D. Monte Carlo tests for associations between disease and alleles at highly poly morphic loci. Ann Hum Genet, 59, 97-105, (1995)).

From the results of the haplotype analysis, a linkage block was estimated using HaploBlockFinder Version 0.6.

[Example 2] Analysis of mu-opioid receptor gene polymorphism in Japanese genome

In order to identify a mu-opioid receptor gene polymorphism, by using the genomic DNA of Japanese normal subjects, the sequence was decoded after PCR amplification for an entire exon region, a part of an intron region, and the 3' and 5' untranslated regions. With regard to the entire exon region and a part of an intron region, a polymorphism analysis was carried out for a region shown with 7 lines in the lower portion of the genomic schematic view of Fig. 1. Further, with regard to A118G, IVS2+G691C, a group of SNPs being in complete linkage disequilibrium found in a wide range from intron 3 to around 4 kbp downstream of the 3' UTR, and a dinucleotide repeat polymorphism, IVS3+6113 $(GT)_{11-15}$ (Example 3) and 32-nucleotide repeat polymorphism, IVS3+8761 $(32bp)_{2-17}$ (Example 3), a linkage disequilibrium analysis was carried out based on the polymorphism frequency of normal subject samples, and a D' value among polymorphisms exhibiting significant linkage disequilibrium is shown in Fig. 1 ($P < 0.05$).

In the decoded regions, 38 novel gene polymorphisms in total, i.e., 36 polymorphisms in Table 1 and 2 polymorphisms in Table 3, and three SNPs (A118G, IVS2+G31A and IVS2+G691C), which had been already reported in studies using other races were found on the Japanese genomes (above Table 1).

Among 39 SNPs shown in Table 1 found in Japanese, majority of them, i.e., 37 SNPs were transversion mutations, and 2 sites (IVS2+G691C and TAA+G2395C) were transition mutations. Further, it was confirmed that because all these polymorphisms exhibited Hardy-Weinberg (HW) equilibrium, there was no bias among collected samples. On the Japanese genomes in the present Example, 14 SNPs were confirmed in the 5' untranslated region, however, 24 SNPs which had been reported in the same region before were not found.

Further, with regard to two SNPs, i.e., A118G and IVS2+G691C, their polymorphism frequencies ("Allelic frequency" in Table 1) in the Japanese genomes were 45.3% and 81.9%, respectively, while their polymorphism frequencies which had been reported mainly in the European and American genomes ("Reported Allelic frequency" in Table 1) were 7. 5 to 25.8% and 42.5 to 53.3%, respectively. The two SNPs were found at a significantly high frequency in the Japanese genomes (Hoehe MR, Kopke K, Wendel B, Rohde K, Flachmeier C, Kidd KK, et al. Sequence variability and candidate gene analysis in complex disease: association of mu opioid receptor gene variation with substance dependence. Hum Mol Genet, 9, 2895-908, (2000)).

Further, SNP present in other translated regions such as C17T, which had been reported to be found at a high polymorphism frequency in a study using other races before, was not observed, either. (Hoehe MR, Kopke K, Wendel B, Rohde K, Flachmeier C, Kidd KK, et al. Sequence variability and candidate gene analysis in complex disease: association of mu opioid receptor gene variation with substance dependence. Hum Mol Genet, 9, 2895-908, (2000), Bond C, LaForge KS, Tian M, Melia D, Zhang S, Borg L, et al. Single-nacleotide polymorphism in the human mu opioid receptor gene alters beta-endorphin binding and activity: possible implications for opiate addiction. Proc Natl Acad Sci U S A, 95, 9608-13, (1998), Bergen AW, Kokoszka J, Peterson R, Long JC, Virkkunen M, Linnoila M, et al. Mu opioid receptor gene variants: lack of association with alcohol dependence. Mol Psychiatry, 2, 490-4, (1997), Sander T, Gscheidel N, Wendel B, Samochowiec J, Smolka M, Rommelspacher H, et al. Human mu-opioid receptor variation and alcohol dependence. Alcohol Clin Exp Res, 22, 2108-10, (1998), Gelernter J, Kranzler H, Cubells J. Genetics of two mu opioid receptor gene (OPRM1) exon I polymorphisms: population studies, and allele frequencies in alcohol- and drug-dependent subjects. Mol Psychiatry, 4, 476-83, (1999), Franke P, Wang T, Nothen MM, Knapp M, Neidt H, Albrecht S, et al. Nonreplication of association between mu-opioid-receptor gene (OPRM1) A118G polymorphism and substance dependence. Am J Med Genet, 105, 114-9, (2001), Shi J, Hui L, Xu Y, Wang F, Huang W, Hu G. Sequence variations in the mu-opioid receptor gene (OPRM1) associated with human addiction to heroin. Hum Mutat, 19, 459-60, (2002), Schinka JA, Town T, Abdullah L, Crawford FC, Ordorica PI, Francis E, et al. A functional polymorphism within the mu-opioid receptor gene and risk for abuse of alcohol and other substances. Mol Psychiatry, 7, 224-8, (2002))

Further, what is interesting is that polymorphisms were found at substantially the same time in some of the SNPs found in intron 3 and the 3' untranslated region, and they showed a D' value of 1.000 in the linkage disequilibrium analysis. Then, in order to examine the size of this linkage disequilibrium block, when the number of cases was reduced (5 cases each for 3 genotypes), and the analyzed range was expanded to about 14 kbp downstream from the stop codon (TAA), additional 57 gene polymorphisms were newly found. The polymorphisms found in the 3' untranslated region of a mu-opioid receptor gene and the linkage disequilibrium thereof are shown in the above Table 2.

Among the newly found 57 gene polymorphisms, 24 gene polymorphisms were observed at the same time as the above-mentioned group of polymorphisms being in linkage disequilibrium, and were in complete linkage disequilibrium showing both D' value and $r^2$ value of 1.000 in the linkage disequilibrium analysis (a symbol "*" in Table 2).

[Example 3] Haplotype analysis and linkage disequilibrium analysis for mu-opioid receptor gene polymorphism

Among the polymorphisms found in Example 2, with regard to 10 sites of SNPs with a polymorphism frequency of 5% or higher, a haplotype was estimated using Arlequin program (above Table 5). When a haplotype was estimated, with regard to the group of SNPs showing complete linkage disequilibrium present in a range from intron 3 to the 3' untranslated region among the SNPs with a polymorphism frequency of 5% or higher, TAA+A2109G, which was one of the SNPs, was used as a representative. By using Arlequin program, 34 haplotypes were estimated, and among these, there were 7 haplotypes observed at a high frequency of 3% or higher (Ne. 1 to No. 7 in Table 5). Those with a frequency of 3% or lower (the remaining 27 haplotypes, which corresponds to 16.1% of the total haplotypes) are collectively shown in No. 8.

The G allele of the above-mentioned TAA+A2109G was observed only in the No. 5 haplotype, and some of the other SNPs also had a similar tendency.

Then, based on the obtained haplotype frequency, a linkage disequilibrium analysis was carried out (above Table 6).

In Table 6, among the results of the linkage disequilibrium analysis, a combination of polymorphisms showing a D' value (upper right of the table) being significant and 0.7 or higher is underlined, and a combination with an $r^2$ value (lower left of the table) being significant and 0.5 or higher is represented by a bold and italic form. When the D' value was calculated as defined above, significant linkage disequilibrium was observed in all the combinations of SNPs except for some of the combinations related to IVS3+G5953A or TAA+A2274G (Table 6).

Further, as for the $r^2$ value, which is a more stringent index, significant linkage disequilibrium was observed in three SNPs in an intron and the 3' untranslated region, and one of the three SNPs was TAA+A2109G, which is one of the above-mentioned groups of polymorphisms showing complete linkage disequilibrium (Table 6).

Further, from the results of the haplotype analysis, four linkage blocks were estimated.

In addition, on the mu-opioid receptor gene, several nucleotide repeat polymorphism other than SNPs were newly found. In intron 3, a dinucleotide repeat polymorphism, IVS3+6113 $(GT)_{11-15}$ and 32-nucleotide repeat polymorphism, IVS3+8761 $(32bp)_{2-17}$ were observed (above Table 3).

Although these repeat polymorphisms exhibit great diversity, significant linkage disequilibrium between them and all the 10 SNPs shown in Table 6 was observed ( $P < 0.05$).

[Example 4] Correlation between mu-opioid receptor gene polymorphism and methamphetamine addiction or stimulant-induced psychotic-like symptoms

In order to examine a correlation between mu-opioid receptor gene polymorphisms and methamphetamine addiction or stimulant-induced psychotic-like symptoms, four sites of SNPs (A118G, IVS2+G691C, IVS3+A6151G and IVS3+G8497T), which are representatives of the SNPs in the linkage blocks estimated at a polymorphism frequency of 5% or higher from the results of the linkage disequilibrium analysis and the haplotype analysis in normal subjects (Example 3) were selected.

First, a polymorphism frequency in all the methamphetamine-addicted patients was analyzed, and compared with that of normal subjects (Table 7).

Table 7: Comparison of polymorphism frequencies of mu-opioid receptor gene polymorphisms between normal subjects and methamphetamine-addicted patients

| Locus | Normal subjects | | | | | | | Methamphetamine-addicted patients | | | | | | | | |
|---|---|---|---|---|---|---|---|---|---|---|---|---|---|---|---|---|
| | Genotypic data | | | | Allelic data | | | Genotypic data | | | | | Allelic data | | | |
| | number | | | allelic frequency | number | | | number | | | allelic frequency | P value | number | | | P value |
| A118G | A | 67 | (0.31) | | A | 233 | (0.55) | A | 49 | (0.38) | | | A | 152 | (0.59) | |
| | A/G | 99 | (0.46) | 45.3% | G | 193 | (0.45) | A/G | 54 | (0.43) | 40.6% | | G | 104 | (0.41) | |
| | G | 47 | (0.22) | | | | | G | 25 | (0.19) | | P=0.43 | | | | P=0.23 |
| | total | 213 | | | total | 426 | | total | 128 | | | | total | 256 | | |
| IVS2 +G691C | G | 6 | (0.03) | | G | 84 | (0.18) | G | 12 | (0.09) | | | G | 67 | (0.26) | |
| | G/C | 72 | (0.31) | 81.9% | C | 380 | (0.82) | G/C | 43 | (0.33) | 73.8% | | C | 189 | (0.74) | |
| | | C154 | (0.66) | | | | | C | 73 | (0.57) | | P=0.012 | | | | p=0.011 |
| | total | 232 | | | | 464 | | total | 128 | | | | total | 256 | | |
| IVS3 +A6151G | A | 2 | (0.01) | | A | 27 | (0.08) | A | 1 | (0.01) | | | A | 28 | (0.11) | |
| | G/A | 23 | (0.13) | 92.5% | G | 331 | (0.92) | G/A | 26 | (0.20) | 89.1% | | G | 228 | (0.89) | |
| | G | 154 | (0.86) | | | | | G | 101 | (0.79) | | P=0.21 | | | | P=0.15 |
| | total | 179 | | | total | 358 | | total | 128 | | | | total | 256 | | |
| IVS3 +C8497T | C | 85 | (0.47) | | C | 246 | (0.69) | C | 73 | (0.57) | | | C | 188 | (0.73) | |
| | C/T | 76 | (0.42) | 31.3% | T | 112 | (0.31) | C/T | 42 | (0.33) | 26.6% | | T | 68 | (0.27) | |
| | T | 18 | (0.10) | | | | | T | 13 | (0.10) | | P=0.209 | | | | p-0.21 |
| | total | 179 | | | total | 358 | | total | 128 | | | | total | 256 | | |

significantly different from that of normal subjects by the comparison are shown. The polymorphism frequency of IVS2+G691C in the methamphetamine-addicted patients is 73.8%, which is lower than 81.9% of the normal subjects. Therefore, it was elucidated that the genotype is significantly different (P = 0.023, chi-square test), and even in the comparison of allelic frequencies, C allele occurred at a high frequency, which is significantly different from normal subj ects (P = 0 . 073, chi-square test).

Further, also in IVS3+A8497G, it was confirmed that there is a tendency in that the ratio of G allele is higher in the methamphetamine-addicted patients than that of the normal subjects.

Subsequently, as for the above-mentioned four SNPs, from the information of gene polymorphisms of the methamphetamine-addicted patients, a haplotype was estimated using Arlequin program (Table 8), and a linkage disequilibrium analysis was carried out based on the resulting haplotype frequency, which was compared with that of normal subjects (Table 9).

Table 8: Comparison of haplotype frequencies of mu-opioid receptor gene polymorphisms between normal subjects and methamphetamine-addicted patients

| | Control | MAP dependent | Haplotype | | | |
|---|---|---|---|---|---|---|
| | frequency | frequency | A118G | IVS2 +G691C | IVS3 +A6151G | IVS3 +C8497T |
| 1 | 1.4 % | | A | C | A | C |
| 2 | 0.2% | | A | C | A | T |
| 3 | 10.9% | 11.0% | A | C | G | C |
| 4 | 24.3% | 23.2% | A | C | G | T |
| 5 | 5.7% | 10.9% | A | G | A | C |
| 6 | 9.2% | 12.7% | A | G | G | C |
| 7 | 1.7% | 1.5% | A | G | G | T |
| 8 | 40.0% | 37.8% | G | C | G | C |
| 9 | 4.9% | 1.8% | G | C | G | T |
| 10 | 0.3% | | G | G | A | T |
| 11 | 1.5% | 0.9% | G | G | G | C |

Table 9: Comparison of linkage disequilibrium of mu-opioid receptor gene polymorphisms between normal subjects and methamphetamine-addicted patients

D'

r²

| Locus | A118G | IVS2 +G691C | IVS3 +A6151G | IVS3 +C8497T |
|---|---|---|---|---|
| A118G | | 0.795<br>0.897 | 0.866<br>1.000 | 0.633<br>0.794 |
| IVS2 +G691C | 0.125<br>0.195 | | 0.746<br>1.000 | 0.775<br>0.739 |
| IVS3 +A6151G | 0.054<br>0.084 | 0.201<br>0.346 | | 1.000<br>1.000 |
| IVS3 +C8497T | 0.159<br>0.156 | 0.062<br>0.071 | 0.037<br>0.044 | |

In Table 9, among the results of the linkage disequilibrium analysis, a combination of polymorphisms showing a D' value (upper right of the table) being significant and 0.7 or higher is underlined. In the respective combinations of the table, the upper numerical values indicate the results of the linkage disequilibrium analysis for the genomes of the normal subjects and the lower numerical values indicate the results of the linkage disequilibrium analysis for the genomes of the methamphetamine-addicted patients.

Table 9 shows the results of the linkage disequilibrium analysis of mu-opioid receptor gene polymorphisms between normal subjects and methamphetamine-addicted patients, and a combination of polymorphisms showing a D' value (upper right of the table) being significant and 0.7 or higher is represented by a dark-gray color. In the respective combinations of Table 9, the upper numerical values indicate the results for the genomes of the normal subjects and the lower numerical values indicate the results for the genomes of the methamphetamine-addicted patients. Further, in all the combinations in the linkage disequilibrium analysis in the four SNPs, the D' values exhibited a significant value, however, any of the $r^2$ values did not exhibit a significant value (Fig. 9).

Although a significant difference was not observed (P = 0.40) with regard to the haplotype frequencies between the normal subjects and the methamphetamine-addicted patients, in the No. 5 and the No. 6 haplotypes in Table 8, the polymorphism frequency of the methamphetamine-addicted patients showed a tendency of being higher compared with that of the normal subjects. The difference between the No. 5 and the No. 6 haplotypes was only a difference in the allele type of IVS3+A6151G, which is one of the groups of SNPs showing complete linkage disequilibrium present in a region from intron 3 to the 3' untranslated region.

Accordingly, it can be said that there is a possibility that an additional haplotype analysis may show a useful difference for addiction to other drugs, pain therapy and the like.

[Example 5] Analysis for cases classified according to stimulant-induced psychotic-like symptoms

In the present Example, comparison of polymorphism frequencies of mu-opioid receptor gene polymorphisms between normal subjects and methamphetamine-addicted patients classified according to stimulant-induced psychotic-like symptoms was carried out.

Methamphetamine-addicted patients were classified as described in Example 1 according to the cases of the stimulant-induced psychotic-like symptoms based on the clinical information, and an analysis was carried out. The analysis was carried out for all the four sites of SNPs (A118G, IVS2+G691C, IVS3+A6151G and IVS3+G8497T), which are representatives of the linkage blocks estimated from the linkage disequilibrium analysis in Example 3. Among them, only polymorphisms in which a group with a polymorphism frequency significantly different from that of normal subjects is present are shown in Table 10.

Table 10: Comparison of polymorphism frequencies of mu-opioid receptor gene polymorphisms between normal subjects and methamphetamine-addicted patients classified according to stimulant-induced psychotic-like symptoms

| Locus | | Normal subjects | | | Methamphetamine-addicted palients | | | | | | | | | | | |
|---|---|---|---|---|---|---|---|---|---|---|---|---|---|---|---|---|
| | | | | | group 1 | | | | group 2 | | | | group 3 | | | |
| | | Number of cases | | Polymorphism frequency | Number of cases | | Polymorphism frequency | | Number of cases | | Polymorphism frequency | | Number of cases | | Polymorphism frequency | |
| (1) 1 | | | | | | | Less than 3 years | | | | 3 years or more | | | | | |
| A118G | A | 67 | (0.31) | | 25 | (0.46) | | | 20 | (0.38) | | | | | | |
| | A/G | 99 | (0.46 ) | 45.3% | 24 | (0.44) | 31.5% | P= | 25 | (0.47) | 38.7% | P= | | | | |
| | G | 47 | (0.22) | | 5 | (0.09) | | 0.0404 | 8 | (0.15) | | 0.4691 | | | | |
| | total | 213 | | | 54 | | | | 53 | | | | | | | |
| IVS2 +G691C | G | 6 | (0.03) | | 6 | (0.11) | | | 5 | (0.09) | | | | | | |
| | G/C | 72 | (0.31) | 81.9% | 20 | (0.37) | 70.4% | P = | 19 | (0.36) | 72.6% | P= | | | | |
| | C | 154 | (0.66) | | 28 | (0.52) | | 0.0085 | 29 | (0.55) | | 0.0394 | | | | |
| | total | 232 | | | 54 | | | | 53 | | | | | | | |
| IVS3 +A6151G | A | 2 | (0.01) | | 0 | (0.00) | | | 10.0 | 19 | | | | | | |
| | G/A | 23 | (0.13) | 92.5% | 15 | (0.08) | 86.1 % | P= | 7 | 0.132 | 91.5% | P= | | | | |
| | G | 154 | (0,86) | | 39 | (0.72) | | 0.0269 | 45 | 0.849 | | 0.9060 | | | | |
| | total | 179 | | | 54 | | | | 53 | | | | | | | |
| (2) *2 | | | | | transient | | | | middle | | | | prolonged | | | |
| IVS2 +G691C | G | 6 | (0.03) | | 8 | (0.11) | | | 0 | (0.00) | | | 3 | (0.12) | | |
| | G/C | 72 | (0.31) | 81.9% | 27 | (0.38) | 70.1% | P= | 5 | (0.29) | 85.3% | P= | 8 | (0.31) | 73.1 % | P= |
| | C | 154 | (0.66) | | 37 | (0.51) | | 0.0034 | 12 | (0.71) | | 0.7807 | 15 | (0.58) | | 0,0596 |
| | total | 232 | | | 72 | | | | 17 | | | | 26 | | | |
| (3) Existence of relapse | | | | | not exist | | | | exist | | | | | | | |
| IVS2 +G691C | G | 6 | (0.03) | | 6 | (0.07) | | | 5 | (0.12) | | | | | | |
| | G/C | 72 | (0.31) | 81.9% | 30 | (0.35) | 75.6% | P= | 14 | (0.33) | 71.4% | P= | | | | |
| | C | 154 | (0.66) | | 50 | (0.58) | | 0.1250 | 23 | (0.55) | | 0.0143 | | | | |
| | total | 232 | | | 86 | | | | 45 | | | | | | | |
| (4) Multiple drug abuse | | | | | none | | | | easily accesible drug | | | | heavy | | | |
| IVS2 | G | 6 | (0.03) | | 5 | (0.14) | | | 3 | (0.06) | | | 1 | (0.02) | | |
| | G/C | 72 | (0.31) | 81.9 % | 11 | (0.31) | 70.8% | P= | 15 | (0.31) | 78.6% | P= | 18 | (0.42) | 76.7% | P= |

(continued)

| Locus | Normal subjects | | | | Methamphetamine-addicted palients | | | | | | | | | | | |
|---|---|---|---|---|---|---|---|---|---|---|---|---|---|---|---|---|
| | | | | | group 1 | | | | group 2 | | | | group 3 | | | |
| | Number of cases | | Polymorphism frequency | | Number of cases | | Polymorphism frequency | | Number of cases | | Polymorphism frequency | | Number of cases | | Polymorphism frequency | |
| +G691C | C | 154 | (0.66) | | 20 | (0.56) | | 0.0059 | 31 | (0.63) | | 0.4401 | 24 | (0.56) | | 0.3801 |
| | total | 232 | | | 36 | | | | 49 | | | | 43 | | | |
| IVS3 +C8497T | C | 85 | (0.47) | | 24 | (0.67) | | | 24 | (0.49) | | | 22 | (0.51) | | |
| | C/T | 76 | (0.42) | 31.3% | 6 | (0.17) | 25.0% | P= | 22 | (0.45) | 28.6% | P= | 17 | (0.40) | 29.1 % | P= |
| | T | 18 | (0.10) | | 6 | (0.17) | | 0.0137 | 3 | (0.06) | | 0.6986 | 4 | (0.09) | | 0.9104 |
| | total | 179 | | | 36 | | | | 49 | | | | 43 | | | |

*1: Period of time of the occurrence of delusion or hallucination
*2: Period of duration of delusion or hallucination

The analysis was carried out for all the four sites of SNPs, which are representatives of the linkage blocks estimated from the linkage disequilibrium analysis of normal subjects, and among them, only polymorphisms in which a group with a polymorphism frequency significantly different from that of normal subjects is present are shown in Table 10.

(1) When classification was carried out according to the period of time from the start of the use of a stimulant to the occurrence of delusion or hallucination, in a group in which a symptom occurred within 3 years, the polymorphism frequencies of three sites of SNPs (A118G, IVS2+G691C and IVS3+A6151G) showed a lower value compared with the normal subj ects, and the genotypes were significantly different (P= 0.040, P= 0.0085, P= 0.027, respectively). With regard to IVS2+G691C, also in a group in which delusion or hallucination occurred after a lapse of not less than 3 years from the start of the use of methamphetamine, the genotype was significantly different in the same manner (P = 0.039) compared with the normal subjects, however, a group, in which the period of time up to the occurrence of symptom is shorter, showed a tendency that the genotype is more significantly different.
(2) When classification was carried out according to the period of time from the stop of the use of a stimulant to the disappearance of delusion or hallucination, in a group in which a symptom relatively promptly disappeared within 1 month, the polymorphism frequency of IVS2+G691C showed a lower value, and the genotype was significantly different compared with the normal subjects (P = 0.0034).
(3) Also in the case where classification was carried out in terms of the presence or absence of the relapse, in a group in which relapse was observed, the polymorphism frequency of IVS2+G691C showed a lower value, and the genotype was significantly different compared with the normal subjects (P = 0.014).
(4) When classification was carried out using a multiple drug abuse as an index, and an analysis was carried out, in a group with a history of only methamphetamine abuse, the polymorphism frequencies of two sites of SNPs (IVS2+G691C and IVS3+C8497T) showed a lower value, and the genotypes were significantly different compared with the normal subjects (P = 0.0059, P = 0.014).
In the Japanese samples, a polymorphism present in a translated region was present in exon 1, and was only A118G which changes asparagine to aspartic acid. It is considered that this asparagine is one site among five glycosylation sites present at the N-terminus of the mu-opioid receptor, which is a seven-transmembrane receptor.

In the present Example, in the comparison of polymorphism frequency of A118G between the normal subjects and the methamphetamine-addicted patients, a significant difference was not observed in the same manner as the past reports (Table 7), however, when the methamphetamine-addicted patients were classified according to stimulant-induced psychotic-like symptoms, the polymorphism frequency was significantly different compared with the normal subjects in some of the groups (Table 10).
When the methamphetamine-addicted patients were classified based on the clinical information and an analysis was carried out, in the period of time of the occurrence of delusion or hallucination, the period of duration and the presence or absence of the relapse, a group in which the polymorphism frequency was significantly lower compared with the normal subjects was observed in some of the SNPs. This indicates that a group with a major allele is liable to develop stimulant-induced psychosis caused by methamphetamine (Table 10).
The present invention shows that a gene polymorphism of the mu-opioid receptor, in particular, IVS2+G691C can be considered as one of the useful gene markers associated with vulnerability to methamphetamine addiction and stimulant-induced psychosis. Further, it shows that a difference in some regulatory mechanism for the expression of the mu-opioid receptor due to a gene polymorphism other than a translated region greatly contributes to various individual differences in an action mediated by the mu-opioid receptor.

Industrial Applicability

According to the present invention, a novel mu-opioid receptor gene polymorphism is provided. By using the polymorphism of the present invention, individual differences in sensitivity to a drug can be evaluated. By this method of evaluating sensitivity, an appropriate prescribed amount of a narcotic drug such as morphine can be found.

Sequence listing free text

SEQ ID NO: 18: n represents m-time repeat of ac (existing position: 51)
SEQ ID NO: 27: n represents 11- to 15-time repeat of gt (existing position: 51).
SEQ ID NO: 31: n represents 2- to 17-time repeat of attatcatattatgacatatatcataatatat (existing position: 51) .
SEQ ID NO: 52: n represents in-time repeat of a (existing position: 51).
SEQ ID NO: 58 : n represents a 322-bp sequence or deletion (existing position: 51).
SEQ ID NO: 60: n represents a or deletion (existing position: 51).
SEQ ID NO: 66: n represents m-time repeat of a (existing position: 51).

SEQ 1D NO: 74: n represents m-time repeat of a (existing position: 51).
SEQ ID NO: 81: n represents m-time repeat of ca (existing position: 51).
SEQ ID NO: 83: n represents tctc or deletion (existing position: 51).
SEQ TD NO: 86: n represents insertion of tttc or does not represent anything (existing position: 51).
(SEQ ID NO: 97: n represents m-time repeat of t (existing position: 51).

Preferred embodiments

1. A method of evaluating drug sensitivity, which comprises linking a gene polymorphism in a mu-opioid receptor gene or a haplotype comprising the gene polymorphism to individual drug sensitivity.

2. The method according to item 1, wherein the drug is a mu-opioid receptor function modulator.

3. The method according to item 2, wherein the mu-opioid receptor function modulator is at least one member selected from the group consisting of methamphetamine methylenedioxymethamphetamine, amphetamine, dextroamphetamine, dopamine, morphine, DAGO, codeine, methadone, carfentanil, fentanyl, heroin, cocaine, naloxone, naltrexone, nalorphine, levallorphan, pentazocine, buprenorphine, oxycodone, hydrocodone, levorphanol, etorphine, dihydroetorphine, hydromorphone, oxymorphone, ethanol, methanol, diethyl ether and tramadol.

4. The method according to item 1, wherein the gene polymorphism is at least one polymorphism selected from the group consisting of single nucleotide polymorphisms, insertion polymorphisms, deletion polymorphisms and nucleotide repeat polymorphisms.

5. The method according to item 4, wherein the gene polymorphism is a gene polymorphism shown in Table 4.

6. The method according to item 1, wherein the haplotype is a haplotype shown in Table 5 or 8.

7. A method of determining a type and/or an amount of a drug to be administered to an individual by using as an index, a result evaluated by the method according to any one of items 1 to 6.

8. A method of predicting a side effect of a drug to be administered to an individual by using as an index, a result evaluated by the method according to any one of items 1 to 6.

9. An oligonucleotide comprising a sequence of at least 10 bases containing the 51st base in a nucleotide sequence represented by any of SEQ ID NOS: 1 to 98 or a sequence complementary to this.

10. The oligonucleotide according to item 9, having a length of 10 to 45 bases.

11. An oligonucleotide selected from the group consisting of the nucleotide sequences represented by SEQ ID NOS: 1 to 98 and nucleotide sequences complementary to these.

12. A microarray, in which the oligonucleotide according to any one of items 9 to 11 has been immobilized on a support.

13. A kit for evaluating drug sensitivity including the oligonucleotide according to any one of items 9 to 11 and/or the microarray according to item 12.

## Claims

1. Method of predicting the sensitivity of an individual to a drug associated with the mu-opioid receptor, comprising:

   determining the genotype of said individual by typing two or more of the following gene polymorphisms i) to iv) in the mu-opioid receptor gene: i) the A118G polymorphism as set forth in SEQ ID NO: 15 or a polymorphism contained in the same haplotype block, ii) the G691C polymorphism as set forth in SEQ ID NO: 24 or a polymorphism contained in the same haplotype block, iii) the A6151G polymorphism as set forth in SEQ ID NO: 28 or a polymorphism contained in the same haplotype block, iv) the C8497T polymorphism as set forth in SEQ ID NO: 30 or a polymorphism contained in the same haplotype block; and
   comparing the obtained genotype of the individual with a predetermined genotype linking the gene polymorphisms in the mu-opioid receptor gene to individual drug sensitivity.

2. The method according to claim 1, wherein the drug is a mu-opioid receptor function modulator.

3. The method according to claim 2, wherein the mu-opioid receptor function modulator is at least one member selected from the group consisting of methamphetamine methylenedioxymethamphetamine, amphetamine, dextroamphetamine, dopamine, morphine, DAMGO, codeine, methadone, carfentanil, fentanyl, heroin, cocaine, naloxone, naltrexone, nalorphine, levallorphan, pentazocine, buprenorphine, oxycodone, hydrocodone, levorphanol, etorphine, dihydroetorphine, hydromorphone, oxymorphone, ethanol, methanol, diethyl ether and tramadol.

4. An oligonucleotide comprising a sequence of at least 10 bases containing the 51st base in a nucleotide sequence represented by any of SEQ ID NOS: 15, 24, 28 and 30 or a sequence complementary to this.

5. The oligonucleotide according to claim 4, having a length of 10 to 45 bases.

6. An oligonucleotide selected from the group consisting of the nucleotide sequences represented by SEQ ID NOS: SEQ ID NOS: 15, 24, 28 and 30 and nucleotide sequences complementary to these.

7. A microarray for predicting drug sensitivity according to the method of claims 1 to 3, in which at least two of the oligonucleotides according to any one of claims 4 to 6 have been immobilized on a support.

8. A kit for predicting drug sensitivity according to the method of claims 1 to 3 including at least two of the oligonucleotides according to any one of claims 4 to 6 and/or the microarray according to claim 7.

FIG. 1

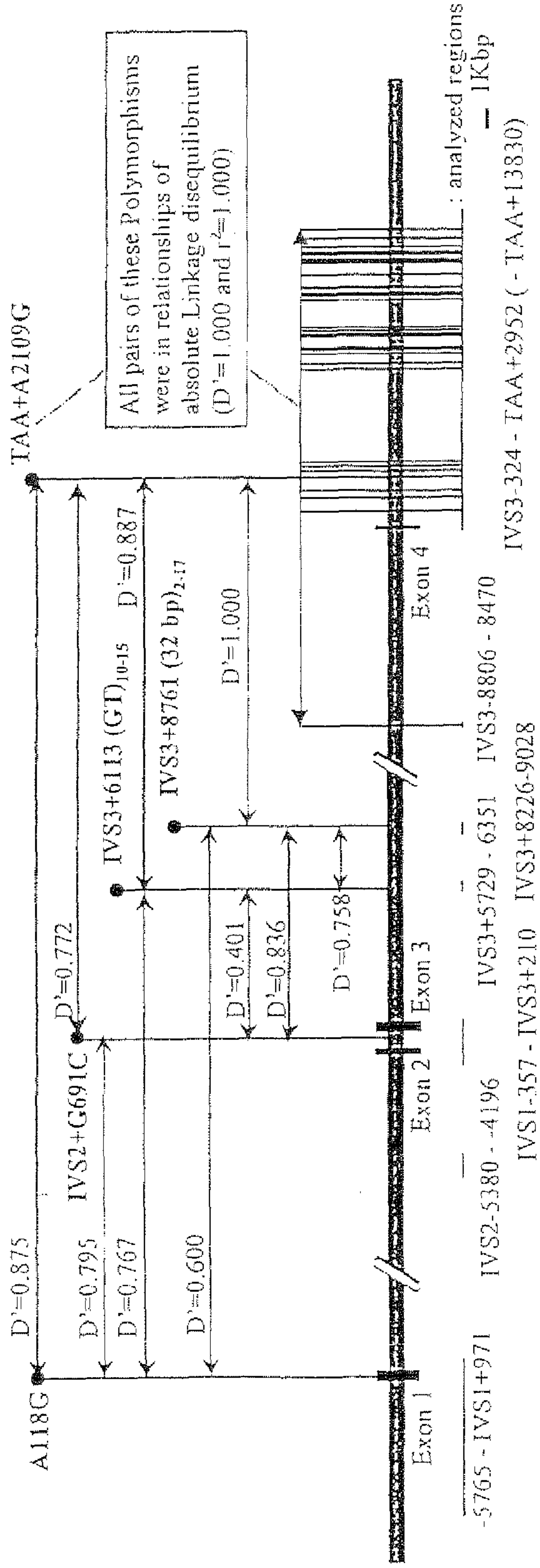

A118G
TAA+A2109G
IVS2+G691C
IVS3+6113 (GT)10-15
IVS3+8761 (32 bp)2-17
D'=0.875
D'=0.795
D'=0.767
D'=0.600
D'=0.772
D'=0.887
D'=1.000
D'=0.401
D'=0.836
D'=0.758
All pairs of these Polymorphisms were in relationships of absolute Linkage disequilibrium (D'=1.000 and r²=1.000)
Exon 1
Exon 2
Exon 3
Exon 4
: analyzed regions
1Kbp
-5765 - IVS1+971
IVS2-5380 - 4196
IVS1-357 - IVS3+210
IVS3+5729 - 6351
IVS3+8226-9028
IVS3-8806 - 8470
IVS3-324 - TAA+2952 ( - TAA+13830)

Europäisches Patentamt
European Patent Office
Office européen des brevets

# EUROPEAN SEARCH REPORT

Application Number
EP 09 15 7733

| Category | Citation of document with indication, where appropriate, of relevant passages | Relevant to claim | CLASSIFICATION OF THE APPLICATION (IPC) |
|---|---|---|---|
| X | HOEHE M R ET AL: "Sequence variability and candidate gene analysis in complex disease: association of mu opioid receptor gene variation with substance dependence" HUMAN MOLECULAR GENETICS, OXFORD UNIVERSITY PRESS, SURREY, vol. 9, no. 19, 1 January 2000 (2000-01-01), pages 2895-2908, XP002990161 ISSN: 0964-6906 * page 2899 - page 2901; table 1 * ----- | 1-8 | INV. C12N15/09 C12M1/00 C12Q1/68 |
| X | LAFORGE K STEVEN ET AL: "Detection of single nucleotide polymorphisms of the human mu opioid receptor gene by hybridization or single nucleotide extension on custom oligonucleotide gelpad microchips: Potential in studies of addiction" AMERICAN JOURNAL OF MEDICAL GENETICS, vol. 96, no. 5, 9 October 2000 (2000-10-09), pages 604-615, XP002529680 ISSN: 0148-7299 * page 609 - page 613; table 1 * ----- | 1-8 | TECHNICAL FIELDS SEARCHED (IPC) C12Q C12N |
| X | TAN ENE-CHOO ET AL: "Mu opioid receptor gene polymorphisms and heroin dependence in Asian populations." NEUROREPORT, vol. 14, no. 4, 24 March 2003 (2003-03-24), pages 569-572, XP009093367 ISSN: 0959-4965 * pages 569,571, column 2; table 1 * ----- -/-- | 1-8 | |

The present search report has been drawn up for all claims

6

| Place of search | Date of completion of the search | Examiner |
|---|---|---|
| Munich | 5 June 2009 | Krüger, Julia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 09 15 7733

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SZETO C Y ET AL: "Association between mu opioid receptor gene polymorphism and Chinese heroin addicts" NEUROREPORT, LIPPINCOTT WILLIAMS & WILKINS, US, vol. 12, no. 6, 8 May 2001 (2001-05-08), pages 1103-1106, XP002990158 ISSN: 0959-4965 * abstract; tables 1,2 * | 1-8 | |
| X | GELERNTER J ET AL: "Genetic of two mu opioid receptor gene (OPRM1) exon I polymorphisms: population studies, and allele frecuencies in alcohol- and drug-dependent subjects" MOLECULAR PSYCHIATRY, BASINGSTOKE, GB, vol. 4, no. 5, 1 January 1999 (1999-01-01), pages 476-483, XP002990159 ISSN: 1359-4184 * table 1 * | 1-8 | TECHNICAL FIELDS SEARCHED (IPC) |
| A | US 2002/006624 A1 (TOWN TERENCE C [US] ET AL) 17 January 2002 (2002-01-17) * paragraphs [0003] - [0006], [0022]; claims 1-20 * | 1-8 | |
| T | IKEDA KAZUTAKA ET AL: "How individual sensitivity to opiates can be predicted by gene analyses." TRENDS IN PHARMACOLOGICAL SCIENCES JUN 2005, vol. 26, no. 6, June 2005 (2005-06), pages 311-317, XP004912366 ISSN: 0165-6147 * the whole document * | 1-8 | |
| | -/-- | | |

The present search report has been drawn up for all claims

| Place of search | Date of completion of the search | Examiner |
|---|---|---|
| Munich | 5 June 2009 | Krüger, Julia |

6

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 09 15 7733

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | IDE ET AL: "Characterization of the 3' untranslated region of the human mu-opioid receptor (MOR-1) mRNA"<br>GENE: AN INTERNATIONAL JOURNAL ON GENES AND GENOMES, ELSEVIER, AMSTERDAM, NL,<br>vol. 364, 30 December 2005 (2005-12-30), pages 139-145, XP005204864<br>ISSN: 0378-1119<br>* the whole document *<br>----- | 1-8 | |
| T | IDE S ET AL: "Linkage disequilibrium and association with methamphetamine dependence/psychosis of mu-opioid receptor gene polymorphisms"<br>PHARMACOGENOMICS JOURNAL, NATURE PUBLISHING GROUP, GB,<br>vol. 6, no. 3, 1 May 2006 (2006-05-01), pages 179-188, XP009117404<br>ISSN: 1470-269X<br>----- | 1-8 | |

TECHNICAL FIELDS SEARCHED (IPC)

The present search report has been drawn up for all claims

| Place of search | Date of completion of the search | Examiner |
|---|---|---|
| Munich | 5 June 2009 | Krüger, Julia |

6

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 09 15 7733

This annex lists the patent family members relating to the patent documents cited in the above-mentioned European search report.
The members are as contained in the European Patent Office EDP file on
The European Patent Office is in no way liable for these particulars which are merely given for the purpose of information.

05-06-2009

| Patent document cited in search report | Publication date | Patent family member(s) | Publication date |
| --- | --- | --- | --- |
| US 2002006624 A1 | 17-01-2002 | US 2004048238 A1 | 11-03-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Uhl GR ; Hall FS ; Sora I.** Cocaine, reward, movement and monoamine transporters. *Mol Psychiatry,* 2002, vol. 7, 21-6
- **Wood PL.** Opioid regulation of CNS dopaminergic pathways: a review of methodology, receptor types, regional variations and species differences. *Peptides,* 1983, vol. 4, 595-601
- **El Daly E ; Chefer V ; Sandill S ; Shippenberg TS.** Modulation of the neurotoxic effects of methamphetamine by the selective kappa-opioid receptor agonist U69593. *J Neurochem,* 2000, vol. 74, 1553-62
- **Hayashi T ; Tsao LI ; Cadet JL ; Su TP.** [D-Ala2, D-Leu5]enkephalin blocks the methamphetamineinduced c-fos mRNA increase in mouse stratum. *Eur J Pharmacol,* 1999, vol. 366, R7-8
- **Smith JW ; Fetsko LA ; Xu R ; Wang Y.** Dopamine D2L receptor knockout mice display deficits in positive and negative reinforcing properties of morphine and in avoidance learning. *Neuroscience,* 2002, vol. 113, 755-65
- **Spielewoy C ; Gonon F ; Roubert C ; Fauchey V ; Jaber M ; Caron MG et al.** Increased rewarding properties of morphine in dopamine-transporter knockout mice. *Eur J Neurosci,* 2000, vol. 12, 1827-37
- **Chefer VI ; Kieffer BL ; Shippenberg TS.** Basal and morphine-evoked dopaminergic neurotransmission in the nucleus accumbens of MOR- and DCR-knockout mice. *Eur J Neurosci,* 2003, vol. 18, 1915-22
- **Lichtman AH ; Sheikh SM ; Loh HH ; Martin BR.** Opioid and cannabinoid modulation of precipitated withdrawal in delta(9)-tetrahydrocannabinol and morphine-dependent mice. *J Pharmacol Exp Ther,* 2001, vol. 298, 1007-14
- **Becker A ; Grecksch G ; Kraus J ; Loh HH ; Schroeder H ; Hollt V.** Rewarding effects of ethanol and cocaine in mu opioid receptor-deficient mice. *Naunyn Schmiedebergs Arch Pharmacol,* 2002, vol. 365, 296-302
- **Berrendero F ; Kieffer BL ; Maldonado R.** Attenuation of nicotine-induced antinociception, rewarding effects, and dependence in mu-opioid receptor knock-out mice. *J Neurosci,* 2002, vol. 22, 10935-40
- **Contarino A ; Picetti R ; Matthes HW ; Koob GF ; Kieffer BL ; Gold LH.** Lack of reward and locomotor stimulation induced by heroin in mu-opioid receptor-deficient mice. *Eur J Pharmacol,* 2002, vol. 446, 103-9
- **Uhl GR ; Sora I ; Wang Z.** The mu opiate receptor as a candidate gene for pain: polymorphisms, variations in expression, nociception, and opiate responses. *Proc Natl Acad Sci U S A,* 1999, vol. 96, 7752-5
- **Hoehe MR ; Kopke K ; Wendel B ; Rohde K ; Flachmeier C ; Kidd KK et al.** Sequence variability and candidate gene analysis in complex disease: association of mu opioid receptor gene variation with substance dependence. *Hum Mol Genet,* 2000, vol. 9, 2895-908
- **Mayer P ; Hollt V.** Allelic and somatic variations in the endogenous opioid system of humans. *Pharmacol Ther,* 2001, vol. 91, 167-77
- **Bond C ; LaForge KS ; Tian M ; Melia D ; Zhang S ; Borg L et al.** Single-nucleotide polymorphism in the human mu opioid receptor gene alters beta-endorphin binding and activity: possible implications for opiate addiction. *Proc Natl Acad Sci U S A,* 1998, vol. 95, 9608-13
- **Bergen AW ; Kokoszka J ; Peterson R ; Long JC ; Virkkunen M ; Linnoila M et al.** Mu opioid receptor gene variants: lack of association with alcohol dependence. *Mol Psychiatry,* 1997, vol. 2, 490-4
- **Sander T ; Gscheidel N ; Wendel B ; Samochowiec J ; Smolka M ; Rommelspacher H et al.** Human mu-opioid receptor variation and alcohol dependence. *Alcohol Clin Exp Res,* 1998, vol. 22, 2108-10
- **Gelernter J ; Kranzler H ; Cubells J.** Genetics of two mu opioid receptor gene (OPRM1) exon I polymorphisms: population studies, and allele frequencies in alcohol-and drug-dependent subjects. *Mol Psychiatry,* 1999, vol. 4, 476-83
- **Franke P ; Wang T ; Nothen MM ; Knapp M ; Neidt H ; Albrecht S et al.** Nonreplication of association between mu-opioid-receptor gene (OPRM1) A118G polymorphism and substance dependence. *Am J Med Genet,* 2001, vol. 105, 114-9
- **Shi J ; Hui L ; Xu Y ; Wang F ; Huang W ; Hu G.** Sequence variations in the mu-opioid receptor gene (OPRM1) associated with human addiction to heroin. *Hum Mutat,* 2002, vol. 19, 459-60

- **Schinka JA ; Town T ; Abdullah L ; Crawford FC ; Ordorica PI ; Francis E et al.** A functional polymorphism within the mu-opioid receptor gene and risk for abuse of alcohol and other substances. *Mol Psychiatry,* 2002, vol. 7, 224-8
- *Ann Hum Genet,* 1995, vol. 59, 97-105
- **Bond C ; LaForge KS ; Tian M ; Melia D ; Zhang S ; Borg L et al.** Single-nacleotide polymorphism in the human mu opioid receptor gene alters beta-endorphin binding and activity: possible implications for opiate addiction. *Proc Natl Acad Sci U S A,* 1998, vol. 95, 9608-13
- **Gelernter J ; Kranzler H ; Cubells J.** Genetics of two mu opioid receptor gene (OPRM1) exon I polymorphisms: population studies, and allele frequencies in alcohol- and drug-dependent subjects. *Mol Psychiatry,* 1999, vol. 4, 476-83